(19) Europäisches Patentamt
European Patent Office
Office européen des brevets

(11) EP 2 550 863 A1

(12) EUROPÄISCHE PATENTANMELDUNG

(43) Veröffentlichungstag:
**30.01.2013 Patentblatt 2013/05**

(21) Anmeldenummer: **11175537.7**

(22) Anmeldetag: **27.07.2011**

(51) Int Cl.:
***A01N 25/24*** (2006.01)
***A61Q 17/02*** (2006.01)
***A61K 9/50*** (2006.01)
***A61K 8/81*** (2006.01)
***A61K 9/16*** (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Bayer Intellectual Property GmbH**
**40789 Monheim (DE)**

(72) Erfinder:
- **Hofmann, Stefan**
  **40764 Lengenfeld (DE)**
- **Selbach, Claudia**
  **42929 Wermelskirchen (DE)**
- **Krüdewagen, Eva Maria**
  **40789 Monheim (DE)**
- **Stanneck, Dorothee**
  **42655 Solingen (DE)**

(74) Vertreter: **BIP Patents**
**c/o Bayer Intellectual Property GmbH**
**Creative Campus Monheim**
**Alfred-Nobel-Straße 10**
**40789 Monheim (DE)**

## (54) Aktivstoffhaltige Partikel auf Polyacrylat-Basis

(57) Die Erfindung betrifft aktivstoffhaltige Partikel auf Polyacrylat-Basis, die an Haare binden, sowie die Verwendung dieser Partikel zur Herstellung von Arzneimitteln, insbesondere für die Veterinärmedizin. Die Partikel weisen eine Größe von maximal 10 $\mu m$ auf und enthalten ungeladenes Polyacrylat und kationisch geladenes Polyacrylat.

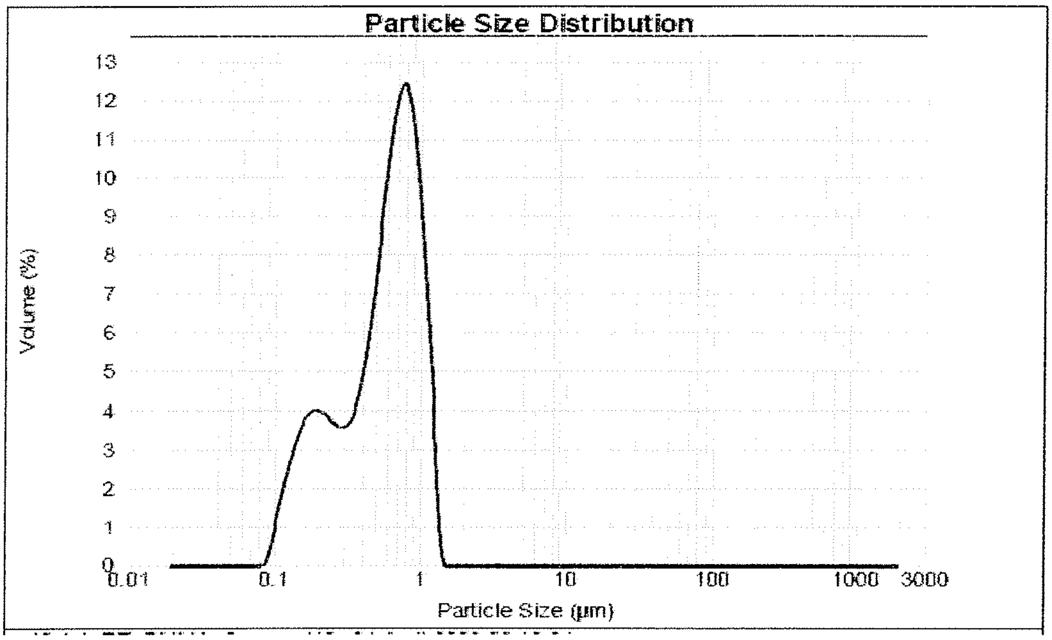


**Fig. 1**

EP 2 550 863 A1

## Beschreibung

Die Erfindung betrifft neue aktivstoffhaltige Partikel auf Polyacrylat-Basis, die an Haare binden, sowie die Verwendung dieser Partikel zur Herstellung von Arzneimitteln, insbesondere für die Veterinärmedizin.

Unter dem Begriff Aktivstoff im Sinne dieser Erfindung sollen im Folgenden sowohl die klassischen pharmazeutischen und insektiziden Wirkstoffe als auch jede Form von tierhalterisch nutzbringenden Stoffen (engl. "beneficial agent") verstanden werden. Er wird synonym mit dem Begriff Wirkstoff verwendet.

Die äußere Applikation von Aktivstoffen ist eine in der Tiermedizin bevorzugte Darreichungsform, die insbesondere bei Formulierungen von Wirkstoffen zum Schutz gegen Ektoparasiten, aber auch von transdermal wirkenden Wirkstoffen sowie solchen Aktivstoffen, die das Verhalten der behandelten Tiere oder auch das von interagierenden Tieren beeinflussen, zum Einsatz kommt. Zu diesem Zweck werden häufig sogenannte Spot-on oder Wipe-On Formulierungen angewendet, wobei der Wirkstoff in flüssiger Form oder als Spray in oder auf das Fell oder die Haut der Tiere aufgebracht wird. Die Wirkungsdauer derartiger Formulierungen ist meist auf wenige Tage oder Wochen, bei repellierenden Aktivstoffen zum Teil auf wenige Stunden beschränkt. In vielen Fällen kann es auch zu Hautreizungen oder ausgedehnten lokalen Entzündungen durch die Wirkstoffe, oder durch Formulierungsbestandteile kommen. Es ist daher von Vorteil, Darreichungsformen zu finden,

- mit denen eine längere Wirkungsdauer erreicht werden kann
- die keine oder nur geringe Hautreizungen verursachen
- die einfach herzustellen sind
- und die die funktionellen oder haptischen Eigenschaften von Tierfell oder Tierhaut nicht beeinträchtigen.

Wie wir nun gefunden haben, besteht eine Möglichkeit dies zu erreichen in kationischen, wirkstoffhaltigen Mikrokapseln geringer Größe, die auf Haut oder Haar des behandelten Tieres appliziert werden und den Wirkstoff kontrolliert und verzögert freisetzen.

Die verzögerte Freisetzung von Aktivstoffen aus ungeladenen und geladenen Mikropartikeln, die auf Haaren oder der Haut appliziert werden, ist im Anwendungsbereich Kosmetik oder Hautpflegeprodukte wohlbekannt. In diesen Anwendungsbereichen können auch die Mikropartikel selbst die Eigenschaften der Haare beeinflussen. Eine länger anhaltende Anwendung im Bereich von Tagen oder Wochen wird bei diesen Anwendungsgebieten jedoch nicht beschrieben und ist auch nicht das Ziel dieser Anwendungen.

In pharmazeutischen Anwendungen ist die Verkapselung von Wirkstoffen in kationische Mikropartikel ebenfalls bekannt und beschrieben. Dabei kommen häufig quaternisierte Dimethylaminoethylmethacrylat Copolymere zum Einsatz (zum Beispiel vom Hersteller Evonik Industries angebotene Polymere mit dem Handelsnamen "Eudragit® RS, RL"). Diese Mikropartikel werden jedoch meist für orale Darreichungsformen in der Pharmazie eingesetzt und sind mit einer Größenordnung von > 30 $\mu$m bis zu 1000 $\mu$m für eine Anwendung am Tierhaar zu groß. Auch führen die bei der Herstellung beschriebenen Methoden nicht zu Mikropartikeln in einer für die erfindungsgemäße Anwendung geeigneten Größenordnung. Die Anwendung von quaternisierten Dimethylaminoethylmethacrylat ist sowohl in Haarpflegeprodukten als auch in transdermalen Therapiesystemen bekannt. Diese Polymere werden dort jedoch nicht in Kombination mit Mikropartikeln eingesetzt.

Eine Vermeidung der Hautreizung durch Wirkstoffe kann prinzipiell dadurch erreicht werden, dass die Wirkstoffe in einem Lösemittel gelöst in Sprayform auf das Fell des Tieres aufgebracht werden. Auch diese Anwendung ist dem Fachmann bekannt und in der Literatur beschrieben. Dadurch wird aber in der Regel keine länger anhaltende Wirkung erreicht.

Im Folgenden ist der Stand der Technik anhand ausgewählter Beispiele detaillierter beschrieben. Durch keine der aufgeführten Methoden und Technologien kann jedoch der Vorteil der vorliegenden Erfindung

- Mikropartikel zur verzögerten Freisetzung von Wirkstoffen bei ausreichender Kleinheit für die Anhaftung an Haare,
- langanhaltende Haftung an Haare durch Belegung der Mikropartikeloberfläche mit kationischen Polymeren,
- keine negative Beeinflussung der funktionellen und haptischen Eigenschaften des Haares, und
- einfache Herstellung über ein Emulgierverfahren

erreicht werden.

Wasserunlösliche, kationische Polymere vom Typ quaternisierter Dimethylaminoethylmethacrylat Copolymere werden als Filmbildner in Tabletten- und Granulatüberzügen eingesetzt, um den Zerfall der Tabletten und die Wirkstofffreisetzung pH-unabhängig zu steuern und zu verzögern. (Evonik Industries: Eudragit® Application Guidelines, 10th Edition, Darmstadt, Germany: Evonik Industries AG, 2008).

Unter quaternisierten Dimethylaminoethylmethacrylat-Copolymeren im Sinne dieser Erfindung wird bevorzugt

eine Gruppe von wasserunlöslichen Polymeren verstanden, die unter dem Handelsnamen Eudragit® RS oder Eudragit® RL der Fa Evonik (Stand 2011) bekannt sind. Dabei handelt es sich um Copolymere der Acrylsäure und Methacrylsäure mit einem niedrigen Anteil quaternisierter Ammoniumgruppen. (Chemische Namen: Poly(ethyl acrylate-co-methyl methacrylate-cotrimethylammonioethyl methacrylate chloride) im Copolymerisationsverhältnis von 1:2:0.1, CAS number: 33434 - 24 - 1, Handelsname Eudragit® RS, beschrieben in Ph. Eur als Ammonio Methacrylate Copolymer, Type B; und Poly(ethyl acrylate-co-methyl methacrylate-cotrimethylammonioethyl methacrylate chloride) Copolymerisationsverhältnis 1:2:0.2, CAS number: 33434 - 24 - 1, Handelsname Eudragit® RL, beschrieben in Ph. Eur. als Ammonio Methacrylate Copolymer, Type A). Diese können als wässrige Dispersion (Eudragit® RS, RL 30D) oder als Granulat verwendet werden (Eudragit® RS, RL PO):

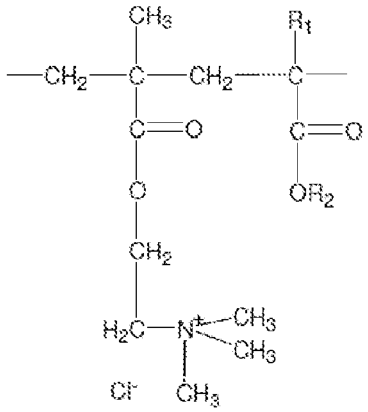

$R_1$ = H, $CH_3$
$R_2$ = $CH_3$, $C_2H_5$

Allgemeine Strukturformel für Copolymere vom Typ "Eudragit® RL, RS"

Das mittlere Molgewicht der Eudragite® Typ RS, RL wird vom Hersteller mit einem Gewichtsmittelwert von $M_w$ = 30.000 g/mol (Eudragit® RS) und $M_w$= 31.000 g/mol (Eudragit® RL) angegeben, die Glasübergangstemperatur wird mit 65 °C (Eudragit® RS) und 70 °C (Eudragit® RL) angegeben. (Evonik Industries: Eudragit Application Guidelines, 10th Edition, Darmstadt, Germany: Evonik Industries AG, 2008).

Es ist bekannt, dass kationische Polymere an negativ geladenen Grenzflächen, wie Haare und Haut gut haften und darauf Filme bilden können. Dieses Prinzip machen sich Haarfestiger und Haarpflegeprodukte zu Nutze. Auch dafür finden Eudragite® Verwendung. In EP 1092417 ist z.B. der Einsatz von kationischem Eudragit® als filmbildendes Polymerisat beschrieben, das in Shampoos feindispers eingearbeitet werden kann und dem Haar erhöhte Festigkeit und verbesserten Halt verleiht. Als Zusatz werden haarkosmetische Wirkstoffe, wie Vitamine, aber keine pharmazeutischen Wirkstoffe genannt.

Filmbildende Acrylat-Copolymere des Eudragit® Typs werden auch in dermalen und transdermalen Therapiesystemen eingesetzt. JP 03-077820 erläutert den Einsatz einer Flüssigformulierung dieser Polymere in einem geeigneten Lösemittel, bevorzugt Ethanol, das zusätzlich antibakteriell oder entzündungshemmende Agentien enthält. Ebenso ist von Insektenrepellentien als Wirkstoff die Rede. Die Formulierungen werden als Flüssigkeit oder Spray auf die Haut aufgebracht. In WO02/060417 werden die kationischen Methacrylat-Copolymere als Haft- oder Bindemittel für transdermale Therapiesysteme in Anspruch genommen. Die Formulierungen enthalten Weichmacher und pharmazeutische Aktivstoffe. Eine weitere Ausführungsform sind dentale Anwendungen auf der Mundschleimhaut oder für die Behandlung von Zahntaschen. In US 5438076 und EP 0404558 wird der Einsatz von Eudragit® L, RL oder RS in alkoholischer Lösung zusammen mit antibakteriellen Wirkstoffen und Weichmachern beschrieben. Eine länger anhaltende Freisetzung der Wirkstoffe aus den auf der Schleimhaut haftenden Filmen wird festgestellt. Die verringerte Wasserlöslichkeit der Polymermaterialien ist ferner von Vorteil, da die Ablösung durch Speichel verzögert, der biologische Abbau aber dennoch gewährleistet ist. JP 63-130541 beschreibt ein ähnliches Verfahren, in dem das Eudragit® zusammen mit antibakteriellem Wirkstoff und hydrophilen Polymeren (Celluloseether, PVP) in höherwertigen Alkoholen gelöst wird und der Wirkstoff, verglichen mit Zubereitungen ohne kationisches Polymer, länger aus den entstandenen Filmen freigesetzt wird. Der Terminus "langanhaltende Freisetzung/Applikation" bezieht sich in solchen Systemen immer auf eine Zeitspanne im Bereich von Stunden bis zu wenigen Tagen. Die für die erfindungsgemäße Anwendung nötigen Zeiträume können damit nicht erreicht werden.

Mikropartikel, die an Haare oder auf der Haut haften, sind prinzipiell in der Lage, darin enthaltene Wirkstoffe über einen längeren Zeitraum freizusetzen. Dabei ist jedoch die Schwierigkeit zu überwinden, eine längere Anhaftung der Mikropartikel auf den Haaren erreichen zu müssen. Da Haare eine negative Oberflächenladung besitzen, ist es

denkbar, die Anhaftung dadurch zu fördern, dass a) die Partikel kationisiert werden oder b) alternativ die Haare mit kationischen Überzügen ausgestattet werden, um so negativ geladene Mikropartikel an Haare zu binden.

Vorgehensweise b) ist in WO01/87243 dargestellt. Darin wird der Einsatz von PTFE Mikropartikeln in Haarpflegeprodukten beschrieben, deren Anhaftung an Haaren dadurch verbessert wurde, dass der Zubereitung kationische Polymere zugesetzt wurden. Erwähnung finden hier kationisierte Dialkylmethacrylamide. Diese PTFE Partikel verbessern Haareigenschaften. Ein ähnliches Verfahren nutzt WO97/38667. Mikropartikel aus Polystyrol, PMMA und anderen Polymeren von 0,2-1 μm Durchmesser werden auf Haare aufgezogen. Die Kationisierung der Partikel erfolgt durch kationische Polymere oder kationische Tenside, die entweder als Matrixpolymer eingesetzt werden oder als Zusatz den Formulierungen beigemischt werden. Die Partikel dienen der Verbesserung des Haarglanzes. Eine lang andauernde Anhaftung über Wochen wird jedoch nicht erreicht und beschrieben. Diese Systeme dienen ebenfalls nicht zur Applikation von Wirkstoffen.

Alternativ können anionische Mikropartikel mit kationischen Polymeren überzogen werden. US 5753264 beschreibt die Erzeugung von Voremulsionen einer Ölphase, die den Aktivstoff enthält (auf Läuse repellierend wirkende Öle, Vitamine) mit anionischen Tensiden in wässriger Lösung und die anschließende Bildung einer Polymerhülle durch Koazervation mit Chitosan aus saurer, wässriger Lösung durch pH-Verschiebung . Nachvernetzung führt zu kationisch geladenen, sehr feinen Mikropartikeln < 10 μm. Diese Mikropartikel können auf Humanhaar aufgezogen werden. Eine repellierende Wirkung wird im in-vitro Versuch über eine Woche belegt. Es wird jedoch nicht belegt, dass die Wirkungsdauer der verkapselten Emulsionen länger ist, als die der Emulsionen, die unverkapselt aufgetragen werden. In US 0142828 wird dargelegt, dass Aktivstoffe, vorzugsweise Parfümstoffe, aber auch Insektenrepellentien, durch Bildung einer wässrigen Primäremulsion und nachfolgender Polykondensation in Mikropartikel aus Harnstoff/Melamin-Formaldehyd Harze eingebracht werden können. Alternativ werden Komplex-Koazervate aus Gelatine oder Polyacrylatpolymeren genannt. Diese Mikropartikel werden dann in einem zweiten Schritt mit dem kationischen Polymer überzogen. Kationisierte Stärke, Guar, Polysiloxane können hier zum Einsatz kommen, aber auch Polyester werden erwähnt. Diese kationisierten Kapseln von 2-15 μm Durchmesser können auf Haare aufgezogen werden und geben den Inhalt frei. Es wird nachgewiesen, dass, verglichen mit nicht kationsierten Kapseln, wesentlich mehr Aktivstoff auf die Haare aufgebracht und freigegeben werden kann. FR 2801811 beschreibt ein ähnliches Verfahren, bei dem negativ geladene Aktivstoffe enthaltende Mikrokapseln durch Aufziehen eines kationischen Polymers (Polyquaternium Typen) umgeladen und so auf negativ geladene Textilfasern oder auf Haare aufgezogen werden können. Die Verwendung von quaternisierten Dimethylaminoethylmethacrylat Copolymeren (Eudragite® RS, RL) wird in diesen Anmeldungen jedoch nicht erwähnt.

Diese genannten Verfahren benötigen jedoch mehrere Prozessschritte, um die kationischen Mikrokapseln zu erzeugen und sind damit für eine einfache technische Herstellung ungeeignet. Die verwendeten kationischen Polymere müssen zudem wasserlöslich sein. Eudragit® RS/RL sind wasserunlöslich und für die hierin beschriebenen Techniken ungeeignet.

Das Ziel anderer Verfahrensentwicklungen ist es, die Mikrokapseln selbst bei der Herstellung mit kationischer Oberflächenladung zu versehen. WO01/35933 schildert die Erzeugung von Mikrokapseln, indem das zu verkapselnde Material (z.B. Vitamine) zusammen mit dem Hüllpolymer in organischem Lösemittel, das teilweise wasserlöslich sein muss, gelöst wird (meist Ethylacetat). Das bevorzugte Hüllpolymer ist PMMA. Diese Lösung wird in einer Wasserphase dispergiert, die einen Emulgator enthält und mit dem organischen Lösemittel gesättigt wurde. Der Emulsion wird im Lösemittel-Extraktionsverfahren (solvent extraction) das Lösemittel entzogen und Mikropartikel von 3-300 μm entstehen. Die Mikrokapseln tragen jedoch keine Ladung. Eine in der Anmeldung WO01/35933 beschriebene Verfahrensalternative ist deshalb die Verwendung von Eudragit® RS PO als Hüllpolymer, das in einem zweiten Schritt auf die Primärpartikel aufgezogen wird. Dadurch erhalten diese Mikropartikel eine kationische Oberflächenladung. Als Vorteil wird herausgestellt, dass das Verfahren keine chlorierten Kohlenwasserstoffe als Lösemittel benötigt. Die Mikrokapseln können erfindungsgemäß auf Haut oder Haar appliziert werden, sind jedoch mit der genannten Größe ungeeignet für Haaranwendungen. Zur Erzeugung der Voremulsion wird ferner ein externer Emulgator in der äußeren, wässrigen Phase benötigt. Die in der Ölphase gelösten Stoffe können keine selbstemulgierende Wirkung entfalten.

EP 1407753 und EP 1407754 beschreiben einen Prozess, in dem Copolymere von Polyacrylamid und Acrylsäure zusammen mit Melamin-Formaldehydharzen in wässriger Lösung dispergiert werden. Parfümöle werden in diese Lösung eingebracht. Eine Temperaturerhöhung leitet die Polykondensation um die Öltröpfchen herum ein. Durch Hinzufügen von kationischem Polymer während der Reaktionsphase wird dieses in die äußere Schicht der Mikropartikel eingebaut. Ausdrücklich erwähnt wird dabei die Notwendigkeit der chemischen Kompatibilität des Polykondensats mit dem Material der Kapselwand. Diese kationischen Mikropartikel können nun auf Textilien aufgezogen oder in Shampoos für Haar- und Hautanwendungen eingearbeitet werden. Allgemein werden Polyester als Beispiel für kationische Polymergruppen genannt. Eudragite® werden hingegen nicht beschrieben. WO02060399 gibt an, dass kationische Haarpflegemittel oder kationische Polymere, z.B. Polyethylenimin, zusammen mit Wirkstoffen und einem hydrophoben Matrixpolymer aufgeschmolzen werden. Diese Schmelze wird in einer tensidhaltigen, wässrigen Lösung emulgiert und abgekühlt. Die kationischen Mikropartikel besitzen eine Größe von 0,1-0,5 μm und werden in Shampoos eingearbeitet.

Die Partikel haften auf Haaren, wobei die Inhaltsstoffe über mehrere Stunden freigesetzt werden. In EP 0369741 hingegen werden kationisierte, poröse Mikropartikel von vorzugsweise 10-40 μm Durchmesser beschrieben, in deren Poren haarpflegende Substanzen, Sonnenschutzmittel, Parfümöle oder Insektenrepellentien absorbiert werden können. Ein Beladungsgrad von 5-65 % wird angegeben. Die positive Ladung fördert die Adsorption auf keratinischen Materialien. Auf die Möglichkeit der Verwendung von Methacrylaten als Copolymer wird in der Beschreibung hingewiesen. Der Herstellungsprozess ist allerdings kompliziert. Mit Suspensionspolymerisation, mehreren Waschschritten zur Erzeugung der porösen Struktur, Kationisierung durch Protonierung der Partikeloberflächen und Beladung mit Wirkstoff werden mindestens vier Schritte benötigt. Es wird zudem nicht nachgewiesen, dass die Partikel lange auf den Haaren haften bleiben.

WO98/28339 beschreibt alternativ die Suspensionspolymerisation als geeignete Methode, Polymerpartikel von 10-150 μm Durchmesser zu erzeugen, wobei die Polymerpartikel aus hydrophoben Methacrylsäureestern, optional copolymerisiert mit anderen Monomeren, wie Styrol bestehen. Bei der Copolymerisation verwendet werden ferner kationische Monomere, bevorzugt kationisierte (quaternisierte) Dimethylaminoethylmethacrylate (Bestandteil des Eudragits®) und vernetzende Monomere. Die Mikropartikel erhalten so ihre kationische Oberflächenladung. Die Suspensionspolymerisation findet in Gegenwart eines Polymerisationsstabilisators statt. Vorzugsweise werden Polyvinylalkohol oder Celluloseester verwendet. Diese hydroxylgruppenhaltigen Polymere können selbst auch kationische Monomereinheiten besitzen. Der Stabilisator wird bei der Partikelbildung in die Wand des Mikropartikels eingebaut. Dadurch erhalten die Mikropartikel funktionelle Oberflächen, bestehend aus quaternären Alkylammonium Einheiten und Hydroxyl-Gruppen. Der Anteil dieses Polymers an den Mikropartikeln kann 1 bis 25 % betragen. Erfindungsgemäß fördert diese Funktionalisierung die Anhaftung an Fasern, auch an keratinischem Material (Wollfasern). Diese Partikel werden vorzugsweise nachfolgend im Dynamic Swelling Verfahren mit Aktivstoffen beladen. Genannt werden Insektizide, Insektenrepellentien, Parfüme, Pheromone und andere Wirkstoffe. Alternativ kann der Wirkstoff aber auch während der Polymerisation in die entstehenden Mikropartikel eingelagert werden. Die entstehenden Dispersionen sind praktisch frei von Agglomeraten und setzen den Wirkstoff auf den Fasern über mehrere Tage frei. Anwendungen am Haar sind hingegen in dieser Anmeldung nicht beschrieben. Hierfür sind die Partikel auch zu groß. Auch dieses Verfahren erfordert zudem einen komplizierten Polymerisationsschritt und ggf. eine nachträgliche Beladung mit Wirkstoff. Eine Freisetzung über mehrere Wochen ist ebenfalls nicht dargelegt. Die notwendige radikalische Polymerisation kann die Stabilität vieler Wirkstoffe negativ beeinflussen.

Es ist demgegenüber offensichtlich, dass die erfindungsgemäße Ausführungsform eine einfachere Methode darstellt und unmittelbar zu den wirkstoffbeladenen, kationisch geladenen Mikropartikeln in geeigneter Dimension von 0,1-10 μm (bevorzugt 0,1-3 μm) führt. Eudragite® können zudem in unterschiedlichem Anteil in den Partikeln enthalten sein.

Im Prinzip ist auch das Solvent Evaporation Verfahren zur Erzeugung wirkstoffhaltiger Mikropartikel aus quaternisierten Dimethylaminoethylmethacrylat Copolymeren (Handelsname Eudragit® RS,RL) Stand der Technik und hinreichend beschrieben. Diese Verfahren wurden jedoch angewandt und optimiert, um oral zu verabreichende Mikrokapseln mit verlängerter Freisetzungsdauer des Wirkstoffs zu entwickeln. Dabei werden Wirkstoff und Eudragit®-Polymere in organischem Lösemittel gelöst und in eine wässrige Phase oder eine Ölphase dispergiert. Die Wasserphase enthält Emulgatoren, vorzugsweise Polyvinylalkohol oder anionische bzw. nichtionische Tenside. Wird eine Ölphase verwendet, z.B. Paraffinöl, kommen oft Stearate zum Einsatz. Nach Abzug des Lösemittels - es kann dazu auch das Solvent Shift Verfahren angewandt werden - verbleiben die Mikropartikel in der Dispersion. Die Größe liegt meist im Bereich zwischen 10-1000 μm. Als ein Beispiel für ein solches Verfahren sei IL 73597 genannt. Darin wird Tetrahydrofuran (THF) als organisches Lösemittel verwendet. In WO92/01443 wird Eudragit® S 100 und Eudragit® RS 100 zusammen mit vorzugsweise basischem Wirkstoff in Methylenchlorid als Lösemittel gelöst und bevorzugt in Mineralöl, das Magnesiumstearat enthält, dispergiert. Nach Verdampfung des Lösemittels liegen die Mikropartikel feinteilig in der Dispersion vor. Die Partikel besitzen eine Größenordnung von 0-150 μm, < 50 μm wird bevorzugt. Die Wirkstofffreisetzung erfolgt verzögert und annähernd unabhängig vom pH Wert der Umgebung. In Drug Development and Industrial Pharmacy 16(13), 2057-2075 (1990) wird dargelegt, wie Nifedipin in Methylenchlorid zusammen mit den Polymeren Eudragit® RS und RL gelöst wird. Diese Lösung wird in der Wasserphase (Emulgator Polyvinylalkohol) mit Hilfe eines Flügelrührers dispergiert und das Lösemittel verdampft. Die Größe der Partikel hängt von der Rührgeschwindigkeit, dem Polymeranteil, dem Emulgatoranteil, der Viskosität der Ölphase etc. ab. Die Veröffentlichungen hierzu sind zahlreich. Im Journal of Controlled Release, 16, 311-318 (1991) wird der Einfluss der Emulgatoren in der Wasserphase auf die Freisetzung eingekapselter 5-Amino-Salicylsäure (Feststoffdispersion in der $CH_2Cl_2$-Phase) untersucht. In den genannten Literaturstellen werden meist gewöhnliche Rührapparaturen eingesetzt, um die Emulsion zu erzeugen In diesen Anmeldungen und Publikationen wird ein in der Wasserphase gelöster Emulgator zur Herstellung der Emulsion und somit der Mikropartikel als notwendig dargestellt.

Als weitere Beispiele aus der umfangreichen Literatur seien genannt:

- Eudragit® RS and RL (acrylic resins) microcapsules as pH insensitive and sustained release preparations of Ketoprofen; Goto S. et al.; J. Microencapsulation 3(4), 1986, 293-304

- Evaluation of the sustained release properties of Eudragit® RS, RL and S (acrylic resins) microcapsules containing Ketoprofen beagle dogs; Goto S. et al., J Microencapsulation 5(4), 1988, 343-360
- A novel method for preparation of Eudragit® RL microcapsules; Satturwar P.M. et al., J.Microencapsulation 19(4), 2002, 407-413

All diese Verfahren führen jedoch zu Mikropartikeln, die für die Anwendung am Haar zu groß sind. Auch wird in diesen Publikationen nicht dargelegt, dass diese Partikel lang andauernd an Oberflächen anbinden. Ferner wird ein Emulgator in der äußeren, wässrigen Phase zur Herstellung der Emulsionen benötigt. Selbstemulgierende Effekte der Eudragit®-Polymertypen werden nicht beschrieben und kommen in den oben genannten Verfahren nicht zum Tragen.

Ein gänzlich alternatives Verfahren, Wirkstoffpartikel an Tierhaare zu befestigen, wird in WO09/056280 dargelegt. Hierzu werden funktionelle Antikörper eingesetzt. Die Mikropartikel sind mit Carboxylgruppen an der Oberfläche funktionalisiert. Über diese Gruppen werden die Antikörper chemisch in einem Mehrschrittverfahren an die Mikropartikel gebunden. Diese Antikörper besitzen variable Domänen, die spezifisch auf die Haare verschiedener Spezies binden können. Durch die Notwendigkeit, die Antikörper chemisch an die Oberfläche zu binden und die Funktionalität in anwendungstechnisch geforderten Zeiträumen stabil zu erhalten, ist dieses Verfahren jedoch ebenfalls als aufwändig zu betrachten.

Erfindungsgemäß werden keine funktionalen Antikörper zur Vermittlung der Bindung der Mikropartikel auf Tierhaare benötigt.

Durch keine der aufgeführten Methoden und Technologien kann somit der Vorteil der vorliegenden Erfindung - a) die Erzeugung von Mikropartikeln zur verzögerten Freisetzung von Wirkstoffen nach Anhaftung an Haaren, b) die Vermittlung einer lang anhaltenden Haftung an Haaren durch Belegung der Mikropartikeloberfläche mit kationischen Polymeren, c) ausreichende Kleinheit der Mikropartikel, um die Haareigenschaften nicht negativ zu beeinflussen, und d) einfache Herstellung der Mikropartikel über ein Emulgierverfahren - erreicht werden.

Aufgabe der Erfindung war es daher, Darreichungsformen für topische Anwendungen in der Medizin, vorzugsweise Tiermedizin, zu entwickeln, die das folgende komplexe Anforderungsprofil erfüllen können:

a) Aktivstoffe langsam über einen Zeitraum von mehreren Tagen oder Wochen freigeben zu können,
b) Hautreizungen weitgehend vermeiden zu können,
c) funktionelle und haptische Eigenschaften von Fell oder Haut nicht zu beeinträchtigen und die
d) zugleich einfach herstellbar sind.

Diese Aufgabe wird erfindungsgemäß gelöst. Die Erfindung betrifft:

1. Partikel mit einer Partikelgröße d(v,90) von maximal 10 μm enthaltend

   a) ein ungeladenes Polyacrylat und

   b) ein kationisches Polyacrylat, das positiv geladene funktionelle Gruppen trägt, wobei die Partikel

   c) einen oder mehrere Aktivstoffe enthalten und

   d) optional weitere Polymere, Hilfs- oder Zusatzstoffe enthalten können.

2. Partikel gemäß Punkt 1 mit einer Partikelgröße von d(v,90) von 0,1-3 μm.

3. Partikel gemäß einem der Punkte 1 oder 2, worin das kationische Polyacrylat b) ein quaternisiertes Dialkylaminoalkylmethacrylat-Copolymer ist.

4. Partikel gemäß Punkt 3, worin das kationische Polyacrylat b) ein Copolymer aus Acrylsäure(methyl,ethyl)estern, Methacrylsäure(methyl,ethyl)estern und mit Monochlormethan quaternisierten Dimethylamminoethylestern der Methacrylsäure ist (bekannt unter den Handelsnamen Eudragit® RS oder Eudragit® RL).

5. Partikel gemäß einem der vorstehenden Punkte, worin das ungeladene Polyacrylat a) Polymethylmethacrylat ist.

6. Partikel gemäß einem der vorstehenden Punkte, wobei das Mischungsverhältnis des ungeladenen Polyacrylats a) zu dem kationischen Polyacrylat b) 5:95 m/m bis 95:5 m/m, bevorzugt 70:30 m/m bis 95:5 m/m , besonders bevorzugt 80:20 m/m bis 90:10 m/m beträgt.

7. Partikel gemäß einem der vorstehenden Punkte, enthaltend, bezogen auf die Masse der Partikel, 0,1 - 50 Gew.-%, bevorzugt 1 - 20 Gew.-% , besonders bevorzugt 5 - 15 Gew.-% Aktivstoffe.

8. Partikel gemäß einem der vorstehenden Punkte, wobei das ungeladene Polyacrylat ein gewichtsmittleres Molgewicht von 1000 g/mol bis zu 1.000.000 g/mol, vorzugsweise 20.000 bis 600.000 g/mol, besonders bevorzugt 50.000 - 150.000 g/mol aufweist.

9. Partikel gemäß einem der vorstehenden Punkte, enthaltend 0,1-50 Gew.-% bezogen auf die Gesamtmasse der Partikel eines oder mehrerer weiterer Polymere, bevorzugt Polystyrol.

10. Partikel gemäß einem der vorstehenden Punkte, enthaltend einen oder mehrere Weichmacher, Tenside, Kosolventien, wobei deren summierter Anteil bezogen auf die Gesamtmasse der Mikropartikel 0,1 - 40 Gew.-% , bevorzugt 5 - 30 Gew.-%, besonders bevorzugt 5-20 Gew.-% beträgt.

11. Partikel gemäß einem der vorstehenden Punkte, enthaltend als Aktivstoff Flumethrin.

12. Partikel gemäß einem der vorstehenden Punkte, enthaltend als Aktivstoff ein Repellens, bevorzugt Icaridin oder N,N-Diethyl-m-toluamid.

13. Partikel gemäß einem der vorstehenden Punkte, enthaltend als Aktivstoff ein Aryl-Pyrrolidin, bevorzugt N-[[4-[3-(3,5-dichlorophenyl)-3-(trifluoromethyl)-1-pyrrolidinyl]-2-(trifluoro-methyl)phenyl]methyl]-propenamide (CAS-Nr.: 1221692-86-9).

14. Verfahren zur Herstellung der Partikel gemäß einem der vorstehenden Punkte, mit den folgenden Schritten:

(i) Herstellung einer Lösung der Bestandteile a) bis d) in einem nicht oder nur in geringem Maße mit Wasser mischbaren Lösemittel oder Lösemittelgemisch (1),

(ii) Dispergierung der Lösung (i) in einer wässrigen Phase, optional enthaltend Zusatzstoffe und Lösemittel oder Lösemittelgemisch (1) bis zur Sättigung, zur Erzeugung einer feinen, stabilen Emulsion,

(iii) Entfernung des Lösemittels oder Lösemittelgemischs (1) aus den Emulsionströpfchen durch

- I) Verdampfung ("Solvent Evaporation" Verfahren) unter Erhalt einer wässrigen Suspension oder
- II) Sprühtrocknung unter Erhalt eines trockenen Pulvers

15. Mittel enthaltend Partikel gemäß einem der Punkte 1 bis 13

16. Mittel gemäß Punkt 15, worin die Partikel in einem Dispergiermedium dispergiert sind.

17. Verwendung der Partikel gemäß einem der Punkte 1 bis 13 zur Herstellung von Mitteln zur Bekämpfung von Parasiten bei Tieren.

18. Verwendung der Mittel gemäß einem der Punkte 15 oder 16 zur Repellierung von Arthropoden bei Tieren.

19. Mittel gemäß einem der Punkte 15 oder 16 zur Verwendung zur Bekämpfung von Parasiten bei Tieren.

20. Mittel gemäß einem der Punkte 15 oder 16 zur Verwendung zur Repellierung von Arthropoden bei Tieren.

Die erfindungsgemäßen Partikel haben eine Partikelgröße d(v,90) < 10 $\mu$m, bevorzugt d(v,90) < 5 $\mu$m, besonders bevorzugt d(v,90) < 3 $\mu$m, gemessen durch Laserbeugung mit einem Malvern Mastersizer® 2000. Vorzugsweise beträgt die Größe der erfindungsgemäßen Partikel mindestens d(v,90) > 0,1 $\mu$m, besonders bevorzugt d(v,90) > 0,3 $\mu$m, besonders bevorzugt d(v,90) > 0,5 $\mu$m. Soweit nicht anders angegeben sind alle Partikelgrößen d(v,90)-Werte gemessen durch Laserbeugung (Malvern Mastersizer® 2000). Unter d(v,90) wird eine volumenbezogene Partikelgrößenverteilung verstanden, bei der 90 % aller Partikel eine Dimension kleiner oder gleich diesen Wertes aufweisen. Entsprechend sind die Bezeichnungen d(v,50), d(v,10) usw. zu verstehen. Die Messung erfolgt dabei mit der Methode der Laserbeugung mit dem Gerät Mastersizer® 2000 (Dispergiereinheit Hydro 2000G) der Firma Malvern und dem Auswertemodus der Fraunhoferbeugung, da die Brechungsindices der Wirkstoffpartikel nicht bekannt sind. Eine geeignete Menge der Probenlösung wird dabei mit 2-3 ml eines Dispergiermediums (Wasser oder 0,1 % wässrige Dioctylnatriumsulfosuccinat Lösung) unter Rühren vordispergiert. Die Dispersion wird dann unter Rühren (300 UpM) und Umpumpen (900 UpM) in die Dispergiereinheit des Geräts gegeben und vermessen. Die Auswertesoftware gibt die Partikelgröße als d(v,0.5), d(v,0.9)-Werte usw. aus.

Die geladenen und ungeladenen Polyacrylate bilden für die eingebetteten Wirkstoffe eine Matrix. Als ungeladene Polyacrylate im Sinne der Erfindung werden nicht nur Polymere von Acrylsäureestern (Polyacrylate im engeren Sinne) sondern auch solche von Derivaten der Acrylsäureester angesehen. Die Ester sind vorzugsweise Alkylester, wobei die Alkylgruppe vorzugsweise 1 bis 4 Kohlenstoffe enthält; ganz besonders bevorzugt sind Methylester. Die Derivate sind insbesondere Poly(alkyl)acrylsäurealkylester, wobei der Alkylsubstituent der Alkylacrylsäure und die Alkylgruppe des Esters unabhängig voneinander Alkyl mit 1 bis 4 Kohlenstoffatomen sein können; jeweils besonders bevorzugt ist die Methylgruppe. Die Poly(alkyl)acrylsäurealkylester werden besonders bevorzugt eingesetzt und können durch die folgende allgemeine Formel dargestellt werden:

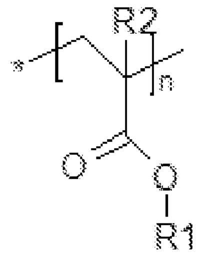

$R^1$ = Alkyl, vorzugsweise mit 1 bis 4 Kohlenstoffatomen, insbesondere $-CH_3$

$R^2$ = Alkyl, vorzugsweise mit 1 bis 4 Kohlenstoffatomen, insbesondere $-CH_3$

Als ungeladenes Polyacrylat für die Matrix ganz besonders bevorzugt ist Polymethacrylsäuremethylester (Polymethylmethacrylat, PMMA),

Als ungeladenes Polyacrylat können in der Matrix auch ungeladene Copolymere der o.g. ungeladenen Polyacrylate eingesetzt werden oder Mischungen verschiedener ungeladener Polyacrylate.

Die Molmassen der ungeladenen Polyacrylate der Matrix, z. B. PMMA, können in großen Bereichen variieren. Molgewichte von $M_w$ = 1000 g/mol bis 1.000.000 g/mol (Gewichtsmittel) können sinnvollerweise verwendet werden. Besonders geeignet ist jedoch ein mittlerer Bereich des Molgewichts von 20.000 - 600.000 g/mol und hier wiederum besonders bevorzugt 50.000-150.000 g/mol. Polyacrylat-Polymere geringeren Molgewichts eignen sich besonders zur Zumischung zur Erniedrigung der Glasübergangstemperatur der Mikropartikel oder zur Erzeugung besonders kleiner Mikropartikel mit d(v,90) < 2μm (hierzu werden bevorzugt Molgewichte $M_w$ < 10.000 g/mol verwendet). Wie bereits weiter oben angegeben, können optional in den Mikropartikeln auch unterschiedliche Anteile weiterer ungeladener Polyalkylacrylsäurealkylester enthalten sein.

Das kationische Polyacrylat trägt positiv geladene funktionelle Gruppen und ist vorzugsweise ein Polyacrylat im engeren Sinne, ein Polymethacrylat oder ein davon abgeleitetes Copolymer. Die Alkylgruppe des Esters sowie gegebenenfalls der Alkylsubstituent der Alkylacrylsäure bedeuten unabhängig voneinander Alkyl mit 1 bis 4 Kohlenstoffatomen, jeweils besonders bevorzugt ist die Methylgruppe. Die positiv geladene Gruppe ist vorzugsweise über die Estergruppe mit dem Polyacrylat-Gerüst verknüpft. Üblicherweise ist eine Amino- bzw. Ammoniumgruppe über eine Alkylkette mit 1 bis 4 Kohlenstoffatomen, vorzugsweise eine Ethylenkette, mit dem Sauerstoff der Estergruppe verbunden. Bei der positiv geladenen Gruppe handelt es sich vorzugsweise um eine dreifach alkylierte und protonierte oder um eine vierfach alkylierte Aminogruppe, wobei die Alkylgruppen unabhängig voneinander 1 bis 4 Kohlenstoffatome, bevorzugt 1 oder 2 Kohlenstoffatome aufweisen. Ganz besonders bevorzugt sind kationische, wasserunlösliche Copolymere aus Dimethylaminoethylmethacrylsäureester, Acrylsäure(ethyl,methyl)ester und Methacrylsäure(ethyl,methyl) ester mit dem Handelsnamen Eudragit® RS bzw. RL (Hersteller und Vertrieb: EVONIK Industries, Stand 2011), bei denen die tertiäre Aminogruppe mit Methylchlorid quaternisiert ist (CAS-Nr 33434 - 24 - 1; Die Polymere vom Typ Eudragit® RL und RS wurden im Vorangehenden bereits detailliert beschrieben). Die kationischen Polyacrylate haben vorzugsweise ein gewichtsmittleres Molgewicht von 20 000 bis 40 000, bevorzugt 25 000 bis 35 000. Das kationische Polyacrylat ist eine separate Komponente der erfindungsgemäßen Partikel, es ist nicht mit den ungeladenen Polyacrylaten der Matrix copolymerisiert.

Überraschenderweise ermöglichen solche kationischen Polymere ein Eigenschaftsprofil, das es ermöglicht, in Kombination mit dem oder den ungeladenen Polyacrylat-Matrixpolymeren alle vier genannten komplexen Anforderungen a) bis d) für die Darreichungsform zu erfüllen. Die Verwendung von kationischen Polymeren, insbesondere der Eudragite® RS, RL, ermöglicht ein einfaches Herstellungsverfahren, durch das kleine Mikropartikel mit kationischer Oberflächenladung erzeugt werden, die aus wässriger Formulierung auf den negativ geladenen Tierhaaren effizient haften können

und die klein genug sind, um die optischen oder haptischen Eigenschaften des Fells nach Trocknung nicht zu beeinträchtigen. Zudem kann eine langdauernde Haftung der Partikel auf dem Tierhaar auch nach der Abtrocknung erreicht werden. Durch die Kombination der unterschiedlichen Polymertypen (Matrixpolymer und kationisches Polyacrylat) in unterschiedlichen Verhältnissen kann die Partikelgröße beeinflusst und eine verzögerte, kontinuierliche und länger anhaltende Freisetzung des oder der Wirkstoffe aus den Partikeln ermöglicht werden Der Anteil an kationischem Polymer kann in weiten Grenzen von 5 - 95 Gew.-%, bevorzugt von 5 - 30 Gew.-%, besonders bevorzugt jedoch von 10-20 Gew.-% bezogen auf den Polymeranteil variieren.

Die erfindungsgemäßen Partikel können auch als Mikrokapseln bezeichnet werden, in die Wirkstoffe eingelagert bzw. verkapselt werden. Darin sind die Wirkstoffe molekulardispers gelöst oder dispers suspendiert. Die Mikropartikel bilden somit ein Wirkstoffreservoir. Die Polymermatrix kann auch weitere Polymere und Zusatzstoffe enthalten. Als weiteres Charakteristikum für die Erfindung ist zu nennen, dass das kationische Polyacrylat und auch weitere Zusatzstoffe mit dem oder den Matrixpolymeren mischbar ist. Weitere Zusatzstoffe und Polymere müssen so gewählt werden, dass keine Phasentrennung innerhalb der Polymermatrix des Partikels eintritt. Der Anteil an den genannten Zusatzstoffen, wie z.B. weiteren Polymeren oder Weichmachern in den Mikropartikeln kann insgesamt bis zu 40 % bezogen auf das Gesamtgewicht der Partikel betragen.

Die **Herstellung der Partikel** kann über verschiedene Verfahren erfolgen. Die erfindungsgemäßen Partikeleigenschaften lassen sich durch das Herstellverfahren beeinflussen. So stellt **das Solvent Evaporation Verfahren** das bevorzugte Verfahren dar, Partikel der erfindungsgemäßen Größe und einer modifizierten Oberfläche zu erhalten. Dieses Verfahren ist in der Literatur vielfach, in verschiedenen Varianten beschrieben. Hierbei werden die Bestandteile der Partikel in einem organischen, nicht mit Wasser mischbaren Lösemittel gelöst, welches dann - meist mit Hilfe eines Emulgators - in einer wässrigen Phase dispergiert wird, so dass zunächst eine Emulsion entsteht. Durch ein Erwärmen dieser Emulsion wird die organische Phase verdampft und den gelösten Bestandteilen die Lösungsgrundlage entzogen. Die Lösemittel können durch geeignete Temperaturführung nahezu vollständig aus den Mikropartikeln entfernt werden. Von den Emulsionströpfchen bleiben die Feststoffe zurück und fallen als Partikel aus. So wird die Emulsion in eine Suspension überführt. Durch Wasch- und Filtrationsschritte können die Partikel aufgereinigt und isoliert werden.

Im erfindungsgemäßen Fall ist eine kationische Oberflächenladung der Partikel für die spätere Anwendung notwendig. Dies wird durch das kationische Polyacrylat erreicht. Das kationische Polyacrylat wird zusammen mit dem Matrixpolymeren, dem oder den Aktivstoffen und gegebenenfalls Zusatzstoffen in der Ölphase gelöst. In der vorliegenden Erfindung kann das Volumenverhältnis des organischen Lösemittels in Bezug auf die Wasserphase in weiten Bereichen variiert werden. So sind Volumenverhältnisse von 10:90 bis 50:50 (organisches Lösemittel : Wasserphase) möglich. Bevorzugt sind Volumenanteile der organischen Phase zu Wasserphase von 20:80 bis 40:60, wodurch bei geeigneten Dispergierbedingungen - z.B. bei Einsatz eines Hochleistungsdispergiergeräts und/oder eines Hochdruckhomogenisators - und Variation des Energieeintrags die Tröpfchengröße der Emulsion und somit letztendlich die Größe der Mikropartikel variiert werden kann. Das organische Lösemittel darf nicht oder nur sehr wenig mit der Wasserphase mischbar sein und muss bei Temperaturen unter dem Siedepunkt des Wassers verdampfbar sein. Dazu sind insbesondere halogenierte Kohlenwasserstoffe und auch Essigsäureethylester (Ethylacetat) in der Lage. Bevorzugt im Sinne der Erfindung sind Dichlormethan, Trichlormethan und Essigsäureethylester.

Die Funktion des Emulgators übernimmt nun erfindungsgemäß das kationische Polyacrylat. Beispielsweise besitzt das bevorzugt verwendete wasserunlösliche kationische Eudragit® RL(RS) in der erfindungsgemäßen Verwendung neben der Kompatibilität zum Matrixpolymer noch bemerkenswert gute emulgierende Eigenschaften, so dass besonders feinteilige Öl-in-Wasser Emulsionströpfchen erzeugt werden können. Dies ist besonders überraschend, da der gängigen Lehrmeinung zufolge ein geeigneter Emulgator zur Erzeugung von Öl-in-Wasser Emulsionen überwiegend in der Wasserphase löslich sein muss. Nach der Entfernung des Lösemittels liegen die wirkstoffhaltigen Mikropartikel in Form einer wässrigen Dispersion vor. Diese besitzen nun auch kationische Oberflächenladung. Dadurch können die Mikropartikel, appliziert als wässrige Dispersionsformulierung, bevorzugt auf die negativ geladenen Oberflächen der Tierhaare gebunden werden. Die Eigenschaften des quaternisierten Dimethylaminoethylmethacrylat Copolymers vom Typ Eudragit® RS, RL führen zu einer besonders guten Haftung an Haaren und sorgen dafür, dass die Mikropartikel auch lange nach Abtrocknung der wässrigen Formuliergrundlage auf den trockenen Tierhaaren haften bleiben. Dadurch wird ein direkter Kontakt der Wirkstoffe mit der Haut vermieden und die hautreizende Wirkung mancher Wirkstoffe kommt nicht zum Tragen. Zudem wird der Wirkstoff in dieser Zeit verzögert aus den Mikrokapseln freigesetzt. Die Freisetzungseigenschaften der Mikropartikel können durch weitere Additive (z.B. Weichmacher, Zusatz anderer Polymertypen, Verhältnis Matrixpolymer/Eudragit®) zusätzlich und in weiten Bereichen variiert werden.

Als Hochleistungsdispergiergerät kann zum Beispiel ein Ultra Turrax® T25 der Firma IKA Werke GmbH & Co. KG zum Einsatz kommen. Ein typischer Einsatzbereich bei der Herstellung der erfindungsgemäßen Mikropartikel ist eine Umdrehungszahl von ca 10.000 UpM bei 1-3 Minuten Einsatzdauer. Als Hochdruckhomogenisator kann zum Beispiel der Typ M110Y der Firma Microfluidics verwendet werden. Im Rahmen der vorliegenden Erfindung wurde bei der Herstellung der Mikropartikel standardmäßig mit einem Druck (Durchflussdruck) von 500 bar und einer Porengröße der Interaktionskammern von 200 und 100 $\mu$m gearbeitet.

Als weiteres, weniger bevorzugtes Verfahren für die Herstellung erfindungsgemäßer Partikel kann die **Sprühtrocknung** genannt werden. Hierzu wird wie bei der Emulsion Evaporation Methode verfahren und die Partikelbestandteile nach dem Lösen in eine Emulsion überführt. Diese kann mittels Zweistoffdüse versprüht und getrocknet werden.

Weniger bevorzugt, aber auch möglich ist eine nachträgliche Beladung der Partikel über einen sogenannten **Dynamic Swelling** Prozess. Dazu werden die Placebopartikel (i.e. erfindungsgemäße Partikel, die noch keine Aktivstoffe enthalten) in einem geeigneten Lösemittel dispergiert. Dieses muss den Aktivstoff lösen und die Partikel quellen lassen. Durch diesen Quellungsprozess und getrieben von der Einstellung eines Verteilungsgleichgewichts zugunsten der organischen Polymerphase ist es dem Aktivstoff möglich, in die Partikel hinein zu diffundieren. Durch den langsamen Entzug des Lösungsmittels nimmt die Quellung der Partikel ab und der Aktivstoff verbleibt in den Mikrokapseln.

Vorzugsweise verwendet werden **Aktivstoffe**, die äußerlich appliziert werden können. Beispielhaft seinen genannt Aktivstoffe aus der Gruppe der Insektizide, Parasitizide, Akarizide, Fungizide, der Repellentien, Dermatika, oder auch verhaltensmodifizierend wirkende Aktivstoffe. Zu solchen verhaltensmodifizierenden Aktivstoffen zählen z.B. Pheromone oder ähnliche, mit dem Fortpflanzungsverhalten in Zusammenhang stehende Geruchsstoffe.

Grundsätzlich können alle für den Fachmann denkbaren, für äußere Applikation geeigneten Aktivstoffe erfindungsgemäß eingesetzt werden. Die im folgenden genannten Aktivstoffe und Aktivstoffgruppen sind somit nur als Beispiele genannt und keinesfalls einschränkend zu werten.

Bevorzugt eingesetzt werden Aktivstoffe gegen Ektoparasiten bei Tieren und Menschen, insbesondere solche mit insektizider und/oder akarizider Wirkung.

Als eine bevorzugte Wirkstoffgruppe seien die Pyrethrine genannt, sowie die **Pyrethroide**, zum Beispiel: Fenvalerate [α-(p-Cl-phenyl)-isovaleriansäure-α-cyano-3-phenoxy-benzylester], Flumethrin [3-[2-(4-Chlorphenyl)-2-chlorvinyl]-2,2-dimethyl-cyclo-propancarbonsäure-(α-cyano-4-fluor-3-phenoxy)-benzylester] und seine Enantiomere sowie Stereoisomere, Cyfluthrin [2,2-Dimethyl-3-(2,2-dichlorvinyl)-cyclopropancarbonsäure-(α-cyano-4-fluor-3-phenoxy)-benzylester], Permethrin [3-Phenoxybenzyl-cis,trans-3-(2,2-dichlorvinyl)-2,2-dimethylcyclopropancarboxylat], Cypermethrin [2,2-Dimethyl-3-(2,2-dichlorvinyl)-cyclopropancarbonsäure-α-cyano-3-phenoxy-benzylester], Deltamethrin [α-Cyano-3-phenoxybenzyl-cis,trans-3-(2,2-dibromvinyl)-2,2-dimethyl-cyclopropancarboxylat], Fluvalinate [2-Cyano-3-phenoxybenzyl-2-(2-chlor-α,α,α,-trifluor-p-toluido)-3-methylbutyrat]. Bevorzugt eingesetzt werden Pyrethroide mit akarazider Wirkung. Besonders bevorzugt sind die α-Cyanopyrethroide, insbesondere die Ester der α-Cyano-3-phenylbenzylalkohole und der 4-Fluoro-α-cyano-3-phenoxybenzylalkohole. Von diesen insbesondere bevorzugt sind Cyfluthrin, β-Cyfluthrin oder insbesondere Flumethrin.

Eine weitere bevorzugte Gruppe von Aktivstoffen sind **Repellentien**. Repellentien (auch Repellens, Repulsivstoff oder Vergrämungsmittel) sind Aktivstoffe, die von einem Organismus üblicherweise über den Geruchssinn wahrgenommen werden und diesen abschrecken, ohne ihn unmittelbar zu töten. Beispielsweise haben Pyrethroide eine repellierende und eine insektizide bzw. akarizide Wirkung. Andere Repellentien haben keine praktisch relevante insektizide oder akarizide Wirkung. Bevorzugt eingesetzt werden Repellentien, die schädliche oder störende Insekten wie Mücken, Fliegen, Flöhe oder Spinnentiere wie Zecken oder Milben von Tieren und Menschen fernhalten. Als bevorzugte Beispiele für die Gruppe der Repellentien seien hier genannt: DEET (Diethylentoluamid), Icaridin, Ethyl-butylacetylaminopropionat (IR3535, MERCK). Die Wirkungsdauer in konventionellen, topisch applizierbaren Handelsformulierungen ist meist auf wenige Stunden beschränkt.

Darüber hinaus bilden die **Aryl-Pyrrolidine** eine weitere bevorzugte Aktivstoffgruppe, die in den erfindungsgemäßen Mikropartikeln verkapselt werden kann. Hier ist insbesondere das **N-[[4-[3-(3,5-dichlorophenyl)-3-(trifluoromethyl)-1-pyrrolidinyl]-2-(trifluoromethyl)phenyl]methyl]-propenamide (CAS-Nr.: 1221692-86-9)** zu nennen.

Aus der Gruppe der Insektizide seien als bevorzugt einsetzbar genannt die im Bereich zur Bekämpfung von ektoparasitierenden Arthropoden verwendeten Insektizide wie Spinosyne, N-Phenylpyrazole, Carbamate, Phosphor- und Phosphonsäureester, Wachstumshemmer, Juvenilhormone sowie Mischungen dieser Wirkstoffe untereinander. Auch können weitere Synergisten zugesetzt werden. Als Synergisten im Sinne dieser Anmeldung werden Verbindungen verstanden, die selbst nicht die gewünschte Wirksamkeit aufweisen, als Mischpartner jedoch zu einer Steigerung der Wirksamkeit der Aktivstoffe führen.

Als Carbamate seien genannt substituierte Phenyl- und Naphthylcarbamate

Als Phosphorsäureester seien bevorzugt genannt die Verbindungen mit den Common Names Phoxim, Fenitrothion, Dichlorvos, Trichlorfon und Malathion.

Alle erfindungsgemäß genannten Aktivstoffe können falls zutreffend sowohl als Stereoisomeren-Gemisch, z. B. als Diastereomerengemisch oder Racemat als auch als angereichertes oder im wesentlichen reines Stereoisomer, z. B. Enantiomer, eingesetzt werden.

Selbstverständlich können in den erfindungsgemäßen Partikeln auch Kombinationen von Wirkstoffen eingesetzt werden.

In den erfindungsgemäßen Partikeln liegen die Aktivstoffe üblicherweise in Konzentrationen von 0,1-50 Gew.-%, bevorzugt 1-20 Gew.-%, besonders bevorzugt 5-15 Gew.-%, jeweils bezogen auf das Gewicht der Partikel vor.

Voraussetzung für den Einarbeitung der Aktivstoffe in Mikropartikel ist die **Kompatibilität mit der Polymermatrix**. Unter Polymermatrix wird im weiteren die Mischung aus dem Matrixpolymer Polyacrylat (bevorzugt PMMA) und dem kationischen Polyacrylat (bevorzugt Eudragit® RL(RS) sowie optional zugesetzten weiteren Polymeren verstanden. Günstig für eine lang anhaltende Freisetzung des Aktivstoffs aus den Mikropartikeln ist es, wenn der Aktivstoff molekulardispers gelöst oder zumindest partikulär amorph, d.h. nicht kristallin, in den Partikeln vorliegt. Besonders bevorzugt ist dabei eine molekulardisperse Verteilung des Aktivstoffs im Matrixpolymer im Sinne einer festen Lösung. Dies kann einmal durch eine grundsätzliche Verträglichkeit im Sinne einer thermodynamischen Mischbarkeit von Wirkstoff und Polymermatrix gegeben sein. Der Vorteil einer solchen Mischbarkeit liegt darin, dass eine rasche Diffusion der Aktivstoffe aus der Matrix an die Oberfläche der Partikel verhindert wird (Prinzip der Phasentrennung). Eine molekulardisperse Verteilung kann z.B. nachgewiesen werden, indem in DSC/DTA Diagrammen keine Schmelzpeaks der Wirkstoffe nachweisbar sind. Eine alternative Methode ist die Analyse durch Röntgendiffraktometrie. Mischbarkeit zeichnet sich bei dieser Methode durch die Abwesenheit von Beugungspeaks aus. Im Sinne der Erfindung soll jedoch nicht ausgeschlossen werden, dass zumindest ein gewisser Teil der Wirkstoffe kristallin in der Polymermatrix eingeschlossen vorliegen kann, wodurch z.B. die Freisetzungsraten zusätzlich modifiziert werden können.

Die Verwendung von Aktivstoffen im Sinne der Erfindung ist jedoch nicht auf solche Aktivstoffe beschränkt, die thermodynamisch stabil mit der Polymermatrix mischbar sind. Bei der Erzeugung der erfindungsgemäßen wirkstoffhaltigen Mikropartikel können auch alle dem Fachmann bekannten Verfahren zum Einsatz kommen, die die **molekulare Mischbarkeit der Wirkstoffe mit der Polymermatrix** erhöhen. Wie in flüssigen Lösungen auch, können dazu ein oder mehrere Lösungsvermittler zum Einsatz kommen. Das kann zum Beispiel erreicht werden durch den Zusatz weiterer Polymere, von Weichmachern, Kosolventien, Netzmitteln (Tensiden) oder auch weiterer Aktivstoffe, die Entmischung, Phasentrennung oder Auskristallisation des Aktivstoffs oder auch anderer Bestandteile in den Mikropartikeln verhindern. Durch Zusatz dieser Stoffe kann auch das Freisetzungsverhalten der Aktivstoffe aus den Mikropartikel beeinflusst werden. Der Zusatz der genannten niedermolekularen Zusatzstoffe senkt in der Regel die Glasübergangstemperatur und erhöht dadurch die Diffusionsgeschwindigkeit der Aktivstoffe in der Matrix, so dass, wenn erwünscht, eine beschleunigte Freisetzung erreicht werden kann.

Als optionaler **Polymerzusatz** kommen generell alle Polymertypen in Frage, die mit dem Matrixpolymer Polyacrylat sowie dem kationischen Polyacrylat und den in den Mikropartikeln eingearbeiteten Aktivstoffen mischbar sind. Vorzugsweise können andere Polyvinylharze wie Polyvinylpyrrolidon, Polyvinylchlorid, Polyvinylpyrrolidon/Polyvinylacetat Copolymere (Handelsname Luviskol), insbesondere aber Polystyrol, zum Einsatz kommen. Auch Derivate polymerer - zumindest teilweise hydrophober - Kohlenhydratverbindungen, z.B Celluloseether, Celluloseester, hydrophobierte Stärke, seien hier genannt, ebenso Polyethylenglycole (Polyoxyethylen, Macrogol, CAS Nr 25322-68-3). Ihr Anteil kann bis zu 50 Gew.-% bezogen auf die Gesamtmasse der Mikropartikel betragen, bevorzugt werden 10-40 Gew.-% verwendet.

Die erfindungsgemäßen Mikropartikel können Weichmacher enthalten. Als **Weichmacher** können alle dem Fachmann bekannten, pharmazeutisch akzeptablen und mit dem Matrixpolymer mischbaren Verbindungen eingesetzt werden, die den gewünschten Effekt, Absenkung der Glasübergangstemperatur, und/oder Steigerung der Mischbarkeit des Aktivstoffs mit dem Matrixpolymeren bewirken. Ebenso kann dadurch die Freisetzungsgeschwindigkeit der Aktivstoffe aus den Mikrokapseln beschleunigt werden. Als Beispiele seien genannt: Phthal- und Therephthalsäureester, Triethylcitrat, Triacetin, Lecithine, Phosphorsäureester, Adipinsäureester, Benzylbenzoat, Tributylacetylcitrat, Ascorbylpalmitat, Ethyloleat und Fettsäureester mehrwertiger Alkohole, wie zum Beispiel des Glycerins und des Propylenglycols (Miglyole). Bevorzugt werden Benzylbenzoat, Tributylacetylcitrat, Triethylcitrat eingesetzt. Es wird üblicherweise so viel Weichmacher zugesetzt, wie nötig ist, um die angestrebte Absenkung der Glasübergangstemperatur und Erhöhung der Freisetzungsgeschwindigkeit zu erreichen. Die nötige Menge kann in weiten Bereichen variieren, jedoch hat sich eine Obergrenze von 40 Gew.-% bezogen auf die Gesamtmasse der Partikel als sinnvoll erwiesen. Vorzugsweise variiert die Einsatzmenge von 10-30 Gew.-%.

Die erfindungsgemäßen Mikropartikel können auch pharmazeutisch verträgliche, mit dem Polymermaterial mischbare **Kosolventien** enthalten, die lösungsvermittelnd und ebenso weichmachend wirken, aber auch den Verteilungskoeffizienten des Aktivstoffs zwischen der Partikelphase und dem Dispergiermedium beeinflussen können. Das Verteilungsgleichgewicht der in diesem Sinne einsetzbaren Kosolventien muss jedoch überwiegend auf der Seite der Polymer/Wirkstoff/Lösemittelphase liegen, um während des Emulgiervorgangs eine Abdiffusion in die äußere, wässrige Phase zu vermeiden. Diese Kosolventien werden üblicherweise in Anteilen von bevorzugt 5 bis 20 Gew.-% bezogen auf den Polymeranteil, eingesetzt. Als geeignete Kosolventien seien beispielsweise pharmazeutisch verträgliche, längerkettige Alkohole, wie n-Butanol, Benzylalkohol oder Ester wie Triacetin, Ethyloleat, Benzylbenzoat genannt. Auch Mischungen der vorstehend genannten Lösungsmittel können als Kosolvenz eingesetzt werden. Selbstverständlich können auch weitere, in diesem Sinne einsetzbare Kosolventien zum Einsatz kommen. Besonders bevorzugt ist Benzylbenzoat.

Die erfindungsgemäßen Mikropartikel können ferner auch pharmazeutisch verträgliche, mit dem Polymermaterial mischbare **grenzflächenaktive Stoffe (Tenside)** enthalten, die ebenfalls lösungsvermittelnd und weichmachend

wirken, aber auch den Verteilungskoeffizienten des Aktivstoffs zwischen der Partikelphase und dem Dispergiermedium beeinflussen können. Das Verteilungsgleichgewicht der in diesem Sinne einsetzbaren **grenzflächenaktiven Stoffe** muss jedoch auch hier überwiegend auf der Seite der Polymer/Wirkstoff/Lösemittelphase liegen, um während des Emulgiervorgangs eine Abdiffusion in die äußere, wässrige Phase zu vermeiden. Es können vorwiegend hydrophobe Tenside und Netzmittel eingesetzt werden, die einen HLB-Wert (Hydrophilic-Lipophilic-Balance Wert, bestimmt nach der Griffin-Methode) von < 8 aufweisen. Diese Tenside und Netzmittel können üblicherweise in Anteilen von bevorzugt 5 bis 20 Gew.-%, bezogen auf den Polymeranteil, eingesetzt werden. Bevorzugt sind nichtionische grenzflächenaktive Stoffe. Als Beispiel seien genannt: Fettalkylpolyethylenglykolether, Alkylphenolpolyethylenglycolether Polyoxyethylen-Fettsäureglyceride, Polyoxyethylen-Fettsäureester jeweils mit niedrigem Oxyethylierungsgrad, Alkylpolyglycoside, Fettsäure-N-methylglucamide, hydrophobere Polysorbate, Sorbitanfettsäureester, Lecithine und Poloxamere mit höherem Polypropylenoxid-Anteil. Selbstverständlich können auch weitere, dem Fachmann bekannte, grenzflächenaktive Stoffe (Tenside) zum Einsatz kommen, die den gewünschten Effekt, Absenkung der Glasübergangstemperatur, und/oder Steigerung der Mischbarkeit des Aktivstoffs mit dem Matrixpolymeren bewirken.

Liegen die Aktivstoffe vorzugsweise molekulardispers in der polymeren Trägermatrix vor, können sie durch Diffusion aus der Matrix in die Umgebung der Mikropartikel freigesetzt werden. Diese Diffusion ist maßgeblich für die lang anhaltende Wirkung auf den Tieren. Die Freisetzungsdauer kann durch **Beeinflussung der Diffusionsgeschwindigkeit der Aktivstoffe** in den Mikropartikeln erreicht werden. Auch hierzu können erfindungsgemäß alle dem Fachmann bekannten Methoden angewandt werden. Im den vorangehenden Abschnitten ist bereits der Zusatz von Weichmachern, Kosolventien, Tensiden und anderen Zusatzstoffen erwähnt, die die Glastemperatur des Matrixpolymers herabsetzen. Als besondere Möglichkeit der Modifikation sei hier zusätzlich die Verwendung von Polymeren unterschiedlichen Molekulargewichts genannt. Der Zusatz von Polymeren niedrigen Molgewichts verringert ebenfalls die Glasübergangstemperatur und kann somit die Diffusionsgeschwindigkeit der Aktivstoffe in der Polymermatrix und somit auch die Freisetzungsgeschwindigkeit modulieren (s. auch Beispiel 4)

Die erfindungsgemäßen Mikropartikel können selbstverständlich alle dem Fachmann bekannten weiteren Zusatzstoffe, die die Stabilität der verkapselten Wirkstoffe oder anderer Aktivstoffe erhöhen oder die Konsistenz der Mikropartikel verbessern, enthalten, sofern sie mit dem Matrixmaterial mischbar sind. Beispielhaft seinen genannt: Antioxidantien, Konservierungsmittel, Füllstoffe.

Zur Einarbeitung in die Mikropartikel besonders geeignete **Antioxidantien** sind die hydrophoberen Vertreter:. Beispielsweise seien genannt: Phenole (Tocopherole, wie auch Vitamin E, Butylhydroxyanisol, Butylhydroxytoluol, Gallensäureester wie zum Beispiel Octyl- und Dodecylgallat, Ascorbinsäurepalmitat sowie weitere geeignete Ester organischer Säuren, Mercaptoverbindungen, zum Beispiel Thioglycerol, Thiomilchsäureester. Die genannten Antioxidantien können in allen Konzentrationsbereichen eingesetzt werden, die ausreichend sind, um die antioxidative Schutzwirkung zu gewährleisten, ein üblicher Konzentrationsbereich ist 0,01-0,1 Gew.-%.

Hydrophobere **Konservierungsmittel** wären zum Beispiel: Benzylalkohol, n-Butanol, Phenol, Cresole, Chlorbutanol, Para-hydroxybenzoesäureester, hier besonders der Propylester. Die genannten Konservierungsmittel können in allen Konzentrationsbereichen eingesetzt werden, die ausreichend sind, um mikrobielle Schutzwirkung zu gewährleisten, ein üblicher Konzentrationsbereich jedoch wäre 0,01-5 Gew.-%.

Die erfindungsgemäßen Mikropartikel werden üblicherweise in eine geeignete **Darreichungsform (Formulierung)** eingebracht. Sie können in Form eines Pulvers oder bevorzugt jedoch als Dispersion, genauer als Suspension, auf das Tier appliziert werden. Unter den Dispersionen sind wässrige Dispersionen bevorzugt. Das beschriebene Herstellungsverfahren liefert bereits einsatzfähige, wässrige Dispersionen als Grundlage für die Formulierung. Dieser Suspension können nun alle dem Fachmann bekannten galenischen Hilfs- und Zusatzstoffe zugesetzt werden, die deren Haltbarkeit, Stabilität und Anwendbarkeit beeinflussen. Die Hilfs- und Zusatzstoffe sollten selbstverständlich mit dem Dispergiermedium kompatibel sein; im Falle des bevorzugten wässrigen Dispergiermediums sollten die Hilfs- und Zusatzstoffe also vorwiegend hydrophil und damit wassermischbar sein. Als Hilfs- und Zusatzstoffe beispielhaft genannt seien **Dispergiermittel**, **Netzmittel (Tenside), Spreitungshilfsmittel, Konservierungsmittel, Antioxidantien, p H-regulierende Agentien, Entschäumer**. Ebenso können **Verdickungsmittel** und Strukturbildner zum Einsatz kommen, um die rheologischen Eigenschaften der Dispersionsformulierungen den Erfordernissen anzupassen Der Zusatz von mischbaren organischen **Lösungsmitteln** zur äußeren Phase kann ein weiteres Mittel sein, um die Freisetzungsprofile der Wirkstoffe aus den Mikropartikeln zu beeinflussen, in dem Sinne, dass dadurch ein bestimmter, fest einstellbarer Anteil der Aktivstoffe bereits gesättigt gelöst in der äußeren Phase der Dispersion vorliegt und somit den erforderlichen Knock-down Effekt für die Parasiten sofort nach Anwendung der Formulierung sicherstellt.

Als Dispergiermedium für die Mikropartikel kommen generell homogene Lösungsmittel und Lösungsmittelgemische mit Zusatzstoffen in Frage, die die Mikropartikel nicht lösen und die Wirkstoffe nicht aus den Mikropartikeln herauslösen. Bevorzugt werden Wasser oder Gemische aus Wasser und wassermischbaren Lösemitteln verwendet, wobei das Mischungsverhältnis des Wassers zu dem wassermischbaren Lösemittel beliebig variieren kann, solange die Mikropartikel nicht gelöst oder stark aufquellen und der Wirkstoff nicht aus den Mikropartikeln herausgelöst wird. Um ein Herauslösen des Aktivstoffs aus den Mikropartikeln zu verhindern, kann das Dispergiermedium auch gelösten

Aktivstoff bis zur Sättigungsgrenze enthalten. Wasserhaltige Dispergiermedien enthalten üblicherweise mindestens 50 Gew.-%, bevorzugt 70 bis 95 Gew.-% Wasser. Die Konzentration der Mikropartikel in diesem Dispergiermedium kann in weiten Bereichen variieren. Partikelkonzentrationen von 1-30 Gew.-% haben sich als geeignet erwiesen. Bevorzugt sind 1-20 Gew.-%, besonders bevorzugt 5-15 Gew.-%.

Als **Dispergiermittel** können alle dem Fachmann bekannten Zusatzstoffe in Frage kommen, die auf den Oberflächen der Mikropartikel adsorbieren oder für eine homogene Verteilung der Mikropartikel in der Dispersionsformulierung sorgen: Bevorzugt zu nennen wären Polyvinylpyrrolidon, Polyvinylalkohol, Celluloseether und -ester sowie Poloxamere (Polyethylenglycol-Polypropylenglycol-Polyethylenglycol Dreiblock-Copolymere). Die genannten Dispergiermittel werden bevorzugt in Konzentrationsbereichen von 0,05 - 3 Gew.-% eingesetzt.

Mögliche Zusatzstoffe aus der Gruppe der **Netzmittel und Tenside** sind bevorzugt hydrophilere, nichtionische und kationische Vertreter mit einem HLB-Wert größer als 8, wie zum Beispiel Fettalkylpolyethylenglycolether, Alkylphenolpolyethylenglycolether, Alkylpolyglycoside, polyoxyethylierte Fettsäureglyceride, polyoxyethylierte Fettsäureester, Fettsäure-N-methylglucamide, Polysorbate, Sorbitanfettsäureester, Poloxamere, polyoxyethylierte Rhizinusölderivate. Bevorzugt sind polyoxyethylierte Sorbitanfettsäureester und Poloxamere zu nennen. Anionische Tenside würden an der Oberfläche der Mikropartikel adsorbieren und die kationische Oberflächenladung reduzieren. Sie sind deshalb weniger geeignet. Geeignete Einsatzkonzentrationen an Dispergiermitteln und Netzmitteln sowie Tensiden werden durch die Partikelkonzentration und die Gesamtoberfläche der Mikropartikel in der Formulierung determiniert und können in weiten Bereichen variieren. Die Konzentration mizellbildender Netzmittel und Tenside wird vorzugsweise so gewählt, dass die kritische Mizellbildungskonzentration (cmc) nicht überschritten wird.

Als **Spreitungshilfsmittel** können bevorzugt wassermischbare Verbindungen eingesetzt werden, wie zum Beispiel nichtionische Tenside und Silicontenside, aber in geringer, noch mit dem Dispergiermittel mischbarer Konzentration, auch ölige Systeme wie Isopropylmyristat, Fettsäureester, Fettalkohole, Adipinsäureester, Triglyceride. Konzentrationsbereiche von 0,01-1 Gew.-% haben sich für Spreitungshilfsmittel häufig als geeignet erwiesen. Diese Konzentrationsangaben, auch die in nachfolgenden Abschnitten, sind jedoch nicht als limitierend zu verstehen und können in Abhängigkeit von Hilfsmittel und weiteren Formulierungsbestandteilen abweichen.

**Konservierungsmittel** können ebenso in den flüssigen Formulierungen enthalten sein. Quaternäre Ammoniumverbindungen eignen sich wegen ihrer kationischen Ladung besonders, da sie bei Adsorption auf der Partikeloberfläche deren positive Ladung nicht reduzieren. Genannt seien hier z.B. Benzalkoniumchlorid, Cetylpyridiniumchlorid. Als Beispiele für weitere, verwendbare Konservierungsmittel seien genannt: aliphatische Alkohole wie Benzylalkohol, Ethanol, Butanol, Phenol, Cresole, Chlorobutanol, Para-hydroxybenzoesäureester, insbesondere die Methyl- und Propylester, Salze oder die freien Säuren der Carbonsäuren, wie Sorbinsäure, Benzoesäure, Milchsäure, Propionsäure. Die Konservierungsmittel sind in den pharmazeutisch üblichen und mikrobiologisch wirksamen Mengen zuzusetzen. Verwendung findende Konzentrationsbereiche sind zum Beispiel 0,01-5 Gew.-%. Sie können sowohl einzeln oder in Kombination mit sogenannten Synergisten eingesetzt werden. Als Synergisten sind zum Beispiel einsetzbar: Zitronensäure, Weinsäure, Ascorbinsäure oder das Natriumsalz der Editinsäure.

Ein Zusatz von **Antioxidantien** kann sinnvoll sein, wenn der in der kontinuierlichen wässrigen Phase gelöste Wirkstoff oder andere Hilfsstoffe oxidationsempfindlich sind. Beispielsweise können zum Einsatz kommen: Sulfite (Natriumsulfit, Natriummetabisulfit), organische Sulfide (Cystin, Cystein, Cysteamin, Methionin, Thioglycerol, Thioglycolsäure, Thiomilchsäure), Phenole, Tocopherole wie Vitamin E, Butylhydroxyanisol, Butylhydroxytoluol, Gallussäureester, z.B. Octyl und Dodecylgallate, organische Säuren (Ascorbinsäure, Citronensäure, Weinsäure, Milchsäure) und deren Salze und Ester. Üblicherweise werden Antioxidantien in Mengen von 0,01-1 Gew.-% zugesetzt.

**Verdickungsmittel und Strukturbildner** sind anorganische Verdickungsmittel wie Bentonite, kolloidale Kieselsäure, Aluminiumstearate, und organische Verdickungsmittel wie Cellulosederivate z.B. Methylcellulose, Carboxymethylcellulose und deren Salze, Hydroxyethylcellulose, Hydroxypropylmethylcellulose 4000, Polyvinylalkohole und deren Copolymere, Polyacrylsäuren (Carbopole), Polyacrylate wie Polyethyl- und -methacrylate, Mischungen von mikronisierter Cellulose und Natriumcarboxymethylcellulose, polymere Kohlenhydrate wie zum Beispiel Xanthan, Alginate, Gummi-Arabicum, Polypeptide wie Gelatine, Polyvinylpyrrolidone, Polyvinylalkohole Stärkederivate, Copolymere aus Methylvinylether und Maleinsäureanhydrid. Abmischungen dieser Substanzklassen können besonders vorteilhaft sein. Meist sind Mengen von 0,01-5 Gew.-% ausreichend, den benötigten Verdickungseffekt zu erzielen.

**pH-Wert regulierende Substanzen** sind pharmazeutisch übliche Säuren oder Basen. Zu den Basen zählen Alkali- oder Erdalkalihydroxyde (z. B. NaOH, KOH), basische Salze wie z.B. Ammoniumchlorid, basische Aminosäuren wie z.B. Arginin, Cholin, Meglumin, Ethanolamine oder auch Puffer wie z.B. Tris(hydroxymethyl)aminomethan, Citronensäure- oder Phosphatpuffer. Zu den Säuren gehören z.B. Salzsäure, Essig-, Wein-, Citronen-, Milch-, Bernstein-, Adipin-, Methansulfon-, Octan-, Linolensäure, Gluconolacton sowie saure Aminosäuren wie z.B. die Asparaginsäure.

Als **Entschäumer** kommen bevorzugt solche auf Silikon-Basis in Frage, beispielsweise Dimeticon oder Simeticon. Hier sind oft bereits sehr geringe Mengen von 0,001-0,01 Gew.-% wirksam.

Der Einsatz von **wassermischbaren Lösungsmitteln** in der wässrigen Phase der Dispersionsformulierung kann sinnvoll sein, um zum Beispiel die Sättigungskonzentration des Aktivstoffs in der kontinuierlichen Phase auf einen

erforderlichen Wert einzustellen. Die Zusätze müssen jedoch mit Sorgfalt gewählt und in der Konzentration begrenzt werden, da die Lösemittel und Kosolventien die Integrität der Mikropartikel nicht kompromittieren und die Wirkstoffe nicht in größeren Mengen aus den Mikropartikeln herauslösen dürfen. Falls wassermischbare Lösemittel zugesetzt werden, finden bevorzugt Einsatzmengen von 5-30 Gew.-% Verwendung. Als Lösungsmittel kommen zum Beispiel in Frage: Physiologisch verträgliche Lösungsmittel wie Alkohole, wie z. B. einwertige Alkanole (z. B. Ethanol oder n-Butanol), mehrwertige Alkohole, wie Glykole (z. B. Ethylenglykol, Propylenglykol, Tetraglycol/Glycofurol), Polyethylenglykole, Polypropylenglykole, Glycerin; aromatisch substituierte Alkohole wie Benzylalkohol, Phenylethanol, Phenoxyethanol; Ester wie Essigester, Butylacetat, Ether wie Alkylenglykolalkylether (z. B. Dipropylenglykolmonomethylether, Diethylenglykolmonoethylether); Ketone wie Aceton, Methylethylketon; Glycerinformal, Solketal (2,2-Dimethyl-4-hydroxymethyl-1,3-dioxolan), N-Methylpyrrolidon, 2-Pyrrolidon, N,N-Dimethylacetamid, Dimethylisosorbit, Lauroglykol, Propylencarbonat, Dimethylformamid, sowie Gemische der genannten Lösungsmittel.

Zur Herstellung der erfindungsgemäßen, flüssigen Formulierungen werden die gewünschten Bestandteile in entsprechenden Mengen miteinander vermischt, z.B. durch den Einsatz konventioneller Rührkessel oder anderer geeigneter Geräte. Falls die Inhaltsstoffe es erfordern, kann unter Schutzatmosphäre oder anderen Methoden des Sauerstoffausschlusses gearbeitet werden.

Die erfindungsgemäßen Mikropartikel, appliziert als wässrige Dispersionsformulierung, adsorbieren auf Grund ihrer positiven Oberflächenladung schnell und bevorzugt auf den negativ geladenen Oberflächen der Tierhaare und bleiben wegen der besonderen Eigenschaften der erfindungsgemäß verwendeten kationischen Polyacrylate über Tage und Wochen auf dem Fell des Tieres haften. Der Wirkstoff kann über diesen gesamten Zeitraum hinweg aus den Mikropartikeln freigesetzt werden und so seine behandelnde oder schützende Wirkung über einen längeren Zeitraum hinweg entfalten. Durch das Anhaften der Mikropartikel auf den Tierhaaren wird der direkte Kontakt mit der Haut weitgehend vermieden und die hautreizende Wirkung vieler Wirkstoffe kommt nicht zum Tragen. Ferner werden durch die geringe Größe der Mikropartikel die optischen und haptischen Eigenschaften des Felles nicht beeinträchtigt. Ein weiterer Vorteil der Erfindung ist, dass Mikropartikel, die unterschiedliche Wirkstoffe enthalten, in einer Dispersionsformulierung gemischt werden können, so dass ansonsten chemisch unverträgliche Wirkstoffe in einer Formulierung gemeinsam appliziert werden können.

Die Applikation der Partikel auf dem Fell erfolgt aus einer wässrigen Suspension zum Beispiel als Spray-, Spot-on-, Pour-on-, Pumpspray-, Aerosol-Spray- oder als Wipe-on-Formulierung. Als Wipe-on Formulierung wird eine Darreichungsform bezeichnet, bei der die Formulierung - vorteilhaft mit einem geeigneten Applikator - über das Fell des Tieres verstrichen oder in das Fell eingearbeitet wird. Bevorzugt ist hier eine Pour-on und eine Wipe-on Formulierung oder eine Darreichung als Pumpspray. Dabei ist die Wipe-on Applikation als besonders bevorzugt zu nennen. Dazu wird anhand des Wirkstoffgehalts der Mikropartikel die benötigte Partikelmenge ermittelt. Über die Feststoffkonzentration in der Mikropartikel-Suspension wird das benötigte Applikationsvolumen errechnet. Für ein vereinfachtes Auftragen wird der wässrigen Dispersion ein Tensid hinzugefügt (z.B. Tween 20,0,01 %). So ist eine bessere Benetzbarkeit des Fells möglich. Die Formulierung wird entweder aufgesprüht oder in das Fell eingerieben. Dazu werden geeignete Applikatoren verwendet.

Die erfindungsgemäßen Formulierungen eignen sich zur vorzugsweise zur äußerlichen Anwendung bei Tieren vorzugsweise bei Warmblütern wie z. B. Vögel oder insbesondere Säugetiere. Dies können Haus- und Nutztiere sowie Zoo-, Labor-, Versuchs- und Hobbytiere sein.

Zu den Nutz- und Zuchttieren gehören Säugetiere wie z.B. Ziegen, Kamele, Wasserbüffel, Esel, Kaninchen, Damwild, Rentiere, Pelztiere wie z.B. Nerze, Chinchilla, Waschbär, sowie insbesondere Rinder, Pferde, Schafe, Schweine.

Zu Labor- und Versuchstieren gehören Mäuse, Ratten, Meerschweinchen, Goldhamster, Hunde und Katzen.

Zu den Hobbytieren gehören Hunde, Katzen und Pferde.

Insbesondere hervorgehoben sei die Anwendung bei Katze, Hund oder Pferd.

Anwendung am Tier schließt erfindungsgemäß auch die Anwendung beim Menschen ein.

Die Anwendung kann sowohl prophylaktisch als auch therapeutisch erfolgen.

Verwendung und Wirkspektrum der erfindungsgemäßen Partikel und der Mittel, die diese enthalten, hängt letzten Endes vom dem enthaltenen Aktivstoff bzw. von den enthaltenen Aktivstoffen ab; die jeweiligen Wirkspektren und Einsatzgebiete sind dem Fachmann grundsätzlich bekannt. Vorzugsweise werden die erfindungsgemäßen Partikel und ihre Formulierungen zur Bekämpfung von Parasiten bei Tieren, insbesondere von Ektoparasiten eingesetzt. Als Parasiten seien Insekten, wie B. Flöhe, Läuse, Mücken, Fliegen etc. und Spinnentiere, wie z. B. von Zecken und Milben genannt. Besonders hervorgehoben sei die Verwendung gegen Flöhe und Zecken.

Die nachfolgenden Beispiele sollen die Erfindung erläutern:

## Beispiele

Beispiel 1

Die Herstellung der Partikel wird mit Hilfe des Emulsion Evaporation Verfahrens durchgeführt. Die Zusammensetzung der Partikel ist nachfolgender Tabelle zu entnehmen.

**Tabelle 1 Einsatzstoffe**

| Einsatzstoffe | Menge |
|---|---|
| Wasser demin. | 160 ml |
| Dichlormethan | 40 ml |
| PMMA | 3,70 g |
| Eudragit® RS 100 | 0,74 g |
| Flumethrin | 0,88 g |

Flumethrin, PMMA (Terez® PMMA 5003, Ter Hell Plastics GmbH, Mw = 94000 g/mol) und Eudragit® RS 100 (Mw = ~ 30000 g/mol; Evonik Industries AG) werden in der organischen Phase (Dichlormethan) gelöst. Mit Hilfe des Ultra Turrax® (T25, IKA® Werke GmbH & Co. KG) wird die organische Phase durch langsame Zugabe in der wässrigen Phase dispergiert (9500 rpm, 2 min.). So wird eine stabile Emulsion erhalten, die dann mit Hilfe eines Hochdruckhomogenisators (Microfluidizer M110Y, Microfluidics, bei 500 bar Durchflussdruck) intensiver homogenisiert wird. Durch leichtes Erwärmen (max. 60 °C) unter Rühren mit Magnetrührer wird die organische Phase entzogen. Die gelösten Bestandteile härten aus und eine Partikelsuspension wird erhalten. Diese kann mit Hilfe einer Rührzelle (Millipore Solvent-resistant Stirred Cell, for 47 mm membranes, Cat No XFUF04701; Millipore GmbH) und geeigneten Filtern (Pall Ultipor N66 / 0,2 µm, Cat No NRG047100; Pall Corporation) filtriert und anschließenden Waschschritten mit Wasser aufgereinigt werden. Danach werden die Partikel charakterisiert:
a) Partikelgrößenanalyse mittels Laserbeugung (Mastersizer® 2000, Malvern Instruments Ltd.) und Rasterelektronenmikroskopie (Sirion 100T, Fa. FEI Company). Ergebnisse siehe Tabelle 2, Fig. 1 und Fig. 2.

**Tabelle 2 Partikelgrößen und analytisch bestimmter Wirkstoffgehalt**

| Probe | D(v,10) [µm] | D(v,50) [µm] | D(v,80) [µm] | D(v,90) [µm] | Wirkstoffgehalt [%] |
|---|---|---|---|---|---|
| 12-PMMA | 0,18 | 0,61 | 0,89 | 1,03 | 15,0 |

b) Wirkstoffgehalt
Der Wirkstoffgehalt wird mit einer HPLC Analyse ermittelt. Hierbei ergibt sich für die Partikel ein Wirkstoffgehalt von 15,0 %.
c) Freisetzung
Die Formulierung wird auf dem Hundefell appliziert, wo die Partikel haften und den Wirkstoff freisetzen. Diese speziellen Freisetzungsbedingungen sind In Vitro schwerlich nach zu stellen. Daher wurde ein Freisetzungsmodell gewählt, welches verlässliche, reproduzierbare Ergebnisse liefert, jedoch lediglich einen Vergleich verschiedener Formulierungen zulässt, da es die komplexen Freisetzungsparameter In Vitro nicht widerspiegeln kann. Als geeignet zeigte sich eine Methanol-Wasser-Mischung im Verhältnis 70/30. Dieses Freisetzungsmedium löst die Partikel weder auf noch quellen sie. Für den Freisetzungsversuch werden die Partikel in 5 ml Freisetzungsmedium dispergiert und bei Raumtemperatur kontinuierlich über einen Zeitraum von sieben Tagen geschüttelt (Stufe 7, waagerechte Probenanordnung; Multi Wrist® Shaker, Lab-Line Instruments). Die Freisetzung beginnt mit einem Burst-Effekt von ~ 10 %. Danach wird der Wirkstoff gleichmäßig freigesetzt. In der logarithmischen Darstellung wird eine Gerade mit einem Bestimmtheitsmaß von $R^2$ = 0,9557 erhalten. Die Freisetzungskurve zeigt, dass nach sieben Tagen (168 h) ca. 45 % des enthaltenen Wirkstoffs freigesetzt wird (Fig. 3)
d) Glasübergangstemperatur
Mit Hilfe der DSC Analyse lässt sich zeigen wie homogen die Partikel aufgebaut sind. Im Fall einer Inhomogenität wären mehrere thermische Reaktionen sichtbar. Sind die Partikelbestandteile von genügender Kompatibilität sollte lediglich eine endotherme Reaktion zu beobachten sein. In diesem Fall handelt es sich um einen Glasübergang. Zudem erweisen sich der Emulgator Eudragit® RS 100 (erste Kurve, $Tg_{Onset}$ = 41 °C) und der Wirkstoff (nicht aufgeführt, da Tg nicht messbar) als Weichmacher, da sie den Glasübergang der wirkstoffbeladenen Partikel (vierte Kurve, $Tg_{Onset}$ = 65 °C)

im Vergleich zu den Placebopartikeln (dritte Kurve, $Tg_{Onset}$ = 92 °C) und zum reinen Polymer (zweite Kurve, $Tg_{Onset}$ = 105 °C) senken (Fig. 4).

Die Messung wurde in einem offenen, 40 μl Aluminium Tiegel durchgeführt (DSC 822$^e$, STAR$^e$ SW 9.20, Mettler Toledo GmbH). Dazu wird die Probe in Temperaturschritten von 10 K/min von 20 °C auf 200 °C erhitzt. In der gleichen Weise wird die Probe von 200 °C auf 20 °C gekühlt und dann nochmals erhitzt.

Beispiel 2

Eine weitere Optimierungs- oder Modifizierungsmöglichkeit besteht in der Auswahl des eingesetzten Matrixpolymers. Das verwendete PMMA kann mit einem weiteren Polymer versetzt werden. So wird wie in Herstellungsvorschrift aus Beispiel 1 verfahren, das Matrixpolymer wird jedoch mit Polystyrol (Mw = ~ 265800 g/mol; PS 158, BASF) versetzt. So sind Abmischungen von 90/10, 80/20 oder auch 60/40 von PMMA und Polystyrol möglich (siehe Tabelle 3). Um eine Mischbarkeit der beiden Polymere zu zeigen, werden Placebo Partikel hergestellt. Eine dieser Formulierungen wird unter Zusatz des Wirkstoffs Flumethrin wiederholt. Der Wirkstoff kann problemlos eingearbeitet werden.

**Tabelle 3 Einsatzstoffe**

| Einsatzstoffe | Menge a) 90/10 | Menge b) 80/20 | Menge c) 60/40 | Menge d) 80/20 mit Wirkstoff |
|---|---|---|---|---|
| Wasser demin. | 80 ml | 80 ml | 80 ml | 80 ml |
| Dichlormethan | 20 ml | 20 ml | 20 ml | 20 ml |
| PMMA | 1,68 g | 1,49 g | 1,12 g | 1,49 g |
| Polystrol (PS158; BASF) | 0,19 g | 0,37 g | 0,75 g | 0,37 g |
| Eudragit® RS 100 | 0,33 g | 0,33 g | 0,33 g | 0,33 g |
| Flumethrin | - | - | - | 0,38 g |

Die sich aus den Formulierungen ergebenden Partikel werden auf ihre Größenverteilung und die Glasübergangstemperatur hin untersucht. Zum Vergleich werden die Glasübergangstemperaturen der reinen Polymere mit aufgenommen (Tabelle 4).

**Tabelle 4 Partikelgrößen und Glasübergangstemperatur**

| Probe | Tg [°C] | D(v,10) [μm] | D(v,50) [μm] | D(v,80) [μm] | D(v,90) [μm] |
|---|---|---|---|---|---|
| PMMA/PS 90/10 | 93,35 | 0,49 | 0,75 | 0,98 | 1,11 |
| PMMA/PS 80/20 | 90,99 | 0,54 | 0,77 | 0,97 | 1,09 |
| PMMA/PS 60/40 | 89,88 | 0,45 | 0,82 | 1,19 | 1,41 |
| PMMA/PS 80/20 mit Flumethrin | 71,06 | 0,37 | 0,72 | 1,07 | 1,28 |
| PS | 85,40 | | | | |
| PMMA | 102,91 | | | | |

Fig. 5 zeigt eine rasterelektronenmikroskopische Aufnahme der Placebo Partikel mit der Abmischung PMMA/PS 60/40.

Beispiel 3

Die Herstellung der Partikel erfolgt wie in Beispiel 1 beschrieben, lediglich das organische Lösungsmittel Dichlormethan wird durch ein anderes Lösemittel ersetzt. Zum Lösen der Partikelbestandteile wird in diesem Beispiel Ethylacetat verwendet. Daten zur Formulierung liegen einmal mit und einmal ohne Wirkstoff (WS) vor.

**Tabelle 5 Einsatzstoffe**

| Einsatzstoffe | Menge a) ohne WS | Menge b) mit WS |
|---|---|---|
| Wasser demin. | 80 ml | 80 ml |

(fortgesetzt)

| Einsatzstoffe | Menge a) ohne WS | Menge b) mit WS |
|---|---|---|
| Ethylacetat | 20 ml | 20 ml |
| PMMA | 1,85 g | 1,85 g |
| Eudragit® RS 100 | 0,33 g | 0,33 g |
| Flumethrin | - | 0,38 g |

Im weiteren Verlauf des Versuchs wird gleich verfahren, jedoch muss die Emulsion zum Verdampfen des Lösemittels auf 75 °C erhitzt werden. Die Partikelgrößenverteilung ist Tabelle 6 zu entnehmen.

**Tabelle 6 Partikelgrößenverteilung**

| Formulierung | D(v,10) [μm] | D(v,50) [μm] | D(v,80) [μm] | D(v,90) [μm] |
|---|---|---|---|---|
| a) ohne WS | 0,26 | 0,55 | 0,95 | 1,31 |
| b) mit WS | 0,45 | 0,77 | 1,08 | 1,26 |

Eine Rasterelektronenmikroskopische Aufnahme zeigt Fig. 6.

Beispiel 4

Die Mikropartikel werden nach der Herstellungsvorschrift aus Beispiel 1 hergestellt. Die Zusammensetzung ist Tabelle 7 zu entnehmen. Als Matrixpolymer werden Polymethylmethacrylate verschiedener Molgewichte (Mw = 2000 bis 600000 Da) verwendet. Auch wird der Wirkstoff Flumethrin gegen ein Aryl-Pyrrolidin Derivat N-[[4-[3-(3,5-dichlorophenyl)-3-(trifluoromethyl)-1-pyrrolidinyl]-2-(trifluoromethyl)phenyl]methyl]-propenamide (CAS-Nr.: 1221692-86-9) ausgetauscht. Auch dieser Wirkstoff lässt sich problemlos in die Mikropartikel einarbeiten.

**Tabelle 7 Einsatzstoffe**

| Einsatzstoffe | Menge |
|---|---|
| Wasser demin. | 160 ml |
| Dichlormethan | 40 ml |
| PMMA (Mw = 2000 - 600000 Da) | 3,70 g |
| Eudragit® RS 100 | 0,65 g |
| Aryl-Pyrrolidin Derivat | 0,77 g |

Tabelle 8 zeigt die erhaltenen Partikelgrößenverteilungen und die Glasübergangstemperaturen. **Tabelle 8 Partikelgrößenverteilung und Glasübergangstemperatur**

| Formulierung mit Matrixpolymer | Tg [°C] | Wirkstoffgehalt [%] | D(v,10) [μm] | D(v,50) [μm] | D(v,80) [μm] | D(v,90) [μm] |
|---|---|---|---|---|---|---|
| PMMA Mw 2000 | 58 | 14,9 | 0,19 | 0,84 | 1,41 | 1,97 |
| PMMA Mw 12000 | 79 | 14,6 | 0,31 | 0,73 | 1,28 | 1,71 |
| PMMA Mw 80000 | 86 | 15,5 | 0,18 | 0,67 | 0,97 | 1,12 |
| PMMA Mw 600000 | 92 | 13,0 | 1,09 | 2,23 | 3,38 | 4,08 |

Die DSC Analysen zeigt Fig. 7 (Methode wie in Beispiel 1 beschrieben).

Mit steigendem Molgewicht ist eine Steigerung der Glasübergangstemperatur zu beobachten.

Beispiel 5

Der Einsatz von Weichmachern kann ebenfalls dazu genutzt werden, die Eigenschaften der Partikel zu modifizieren. Dabei erfolgt die Herstellung wie in Beispiel 1 beschrieben. Der Weichmacher wird mit in der organischen Phase gelöst. Die Zusammensetzung von Formulierungen mit zunehmendem Gehalt an Weichmachern ist nachstehender Tabelle zu entnehmen. In diesem Fall handelt es sich bei dem Weichmacher um Benzylbenzoat, das in der Veterinärmedizin auch als Lösungsmittel bei parenteralen Injektionsformulierungen Verwendung findet.

**Tabelle 9 Einsatzstoffe von Formulierungen verschiedener Weichmacher Konzentration**

| Einsatzstoffe | Menge a)10% | Menge b) 20 % | Menge c) 30 % |
|---|---|---|---|
| Wasser demin. | 80 ml | 80 ml | 80 ml |
| Dichlormethan | 20 ml | 20 ml | 20 ml |
| PMMA | 1,85 g | 1,85 g | 1,85 g |
| Eudragit® RS 100 | 0,33 g | 0,33 g | 0,33 g |
| Benzylbenzoat | 0,24 g | 0,54 g | 0,93 g |

Der Weichmacher kann in verschiedenen Konzentrationen der Formulierung beigemischt werden. So kann die Glasübergangstemperatur variiert werden. Die sich aus der Weichmacherkonzentration ergebenden Glasübergangstemperaturen sind Tabelle 10 zu entnehmen, ebenso wie die Partikelgrößenverteilung.

**Tabelle 10 Partikelgrößenverteilung und Glasübergangstemperaturen**

| Weichmacheranteil %] | Tg [°C] | D(v,10) [μm] | D(v,50) [μm] | D(v,80) [μm] | D(v,90) [μm] |
|---|---|---|---|---|---|
| 10 | 69 | 1,01 | 1,51 | 1,94 | 2,18 |
| 20 | 46 | 1,00 | 1,50 | 1,92 | 2,17 |
| 30 | 26 | 0,96 | 1,44 | 1,85 | 2,08 |

Als Weichmacher können ebenso folgende Substanzen zum Einsatz kommen: Tributylacetylcitrat, Triethylcitrat, Hexamoll® (BASF). Auch diese führen bei steigender Konzentration zu einer Abnahme der Glasübergangstemperatur.

Beispiel 6

Neben der Verkapselung von Insektiziden können auch Repellentien verkapselt werden wie der Wirkstoff Icaridin. Die Wasserlöslichkeit des Wirkstoffs muss jedoch berücksichtigt werden. So wird wie in Beispiel 1 verfahren, aber zusätzlich die Wasserphase mit Wirkstoff gesättigt. Die Zusammensetzung ist nachfolgender Tabelle zu entnehmen.

**Tabelle 11 Einsatzstoffe**

| Einsatzstoffe | Menge |
|---|---|
| Wasser demin. | 160 ml |
| Dichlormethan | 40 ml |
| PMMA | 3,70 g |
| Eudragit® RS 100 | 0,74 g |
| Icaridin | 0,88 g |
| Icaridin (zur Sättigung der Wasserphase) | 2 g |

Die Partikelbestandteile werden in der organischen Phase gelöst. Die wässrige Phase wird mit dem Wirkstoff gesättigt. Die organische Phase wird in der wässrigen Phase mit Hilfe des Ultra Turrax® (9500 rpm, 2 min) dispergiert. Das Lösungsmittel wird durch Erhitzen (45 C) und Rühren mit Magnetrührer entzogen und die Partikelsuspension anschließend mit Wasser gewaschen, abfiltriert und getrocknet. Die Partikelgrößenverteilung enthält Tabelle 12.

**Tabelle 12 Partikelgrößenverteilung**

| Probe | D(v,10) [μm] | D(v,50) [μm] | D(v,80) [μm] | D(v,90) [μm] |
|---|---|---|---|---|
| PMMA-Icaridin | 0,85 | 1,31 | 1,72 | 1,95 |

Die erhaltenen Partikel zeigt Fig. 8.

Beispiel 7

Als weiterer repellierender Wirkstoff kann DEET (N,N-Diethyl-m-toluamid) verkapselt werden. Die Wasserlöslichkeit des Wirkstoffs muss berücksichtigt werden, um eine erfolgreiche Verkapselung zu gewährleisten. Die Herstellung erfolgt wie in Beispiel 6 beschrieben, wobei auch hier die Wasserphase mit Wirkstoff gesättigt wird. Die Partikelgrößenverteilung enthält Tabelle 13.

**Tabelle 13 Partikelgrößenverteilung**

| Probe | D(v,10) [μm] | D(v,50) [μm] | D(v,80) [μm] | D(v,90) [μm] |
|---|---|---|---|---|
| PMMA-DEET | 0,39 | 0,70 | 0,95 | 1,09 |

Beispiel 8

Das Verhältnis der organischen zur wässrigen Phase wurde variiert und zugunsten der organischen Phase optimiert. So ist bei gleichbleibender PMMA Menge bezogen auf die nicht-wässrige Phase eine Erhöhung der erhaltenen Partikelmenge möglich. Die Herstellung der Partikel erfolgt wie unter Beispiel 1 beschrieben. Neben verschiedenen Placeboformulierungen werden auch Verumpartikel generiert. Die Zusammensetzung der einzelnen Formulierungen ist Tabelle 14 zu entnehmen.

**Tabelle 14 Einsatzstoffe**

| Einsatzstoffe | Formulierung | | | | | | |
|---|---|---|---|---|---|---|---|
| | 1 | 2 | 3 | 4 | 5 | 6 | 7 |
| Wasser demin. | 80 ml | 70 ml | 60 ml | 70 ml | 60 ml | 80 ml | 60 ml |
| Dichlormethan | 20 ml | 20 ml | 20 ml | 30 ml | 40 ml | 20 ml | 40 ml |
| PMMA | 1,86 g | 1,86 g | 1,86 g | 2,79 g | 3,72 g | 1,86 g | 3,72 g |
| Eudragit® RS 100 | 0,37 g | 0,37 g | 0,37 g | 0,56 g | 0,74 g | 0,37 g | 0,74 g |
| Flumethrin | - | - | - | - | - | 0,39 g | 0,79 g |

Für die wirkstoffhaltigen Formulierungen ergeben sich für die Partikelgröße folgende Größenverteilungen (Tabelle 15).

**Tabelle 15 Partikelgrößenverteilung**

| Formulierung | D(v,10) [μm] | D(v,50) [μm] | D(v,80) [μm] | D(v,90) [μm] |
|---|---|---|---|---|
| **1** | 1,21 | 1,99 | 2,72 | 3,14 |
| **2** | 1,16 | 1,85 | 2,46 | 2,82 |
| **3** | 1,18 | 1,87 | 2,49 | 2,86 |
| **4** | 1,17 | 1,86 | 2,48 | 2,85 |
| **5** | 1,17 | 1,80 | 2,34 | 2,66 |
| **6** | 0,39 | 0,74 | 1,01 | 1,17 |
| **7** | 0,65 | 1,10 | 1,53 | 1,79 |

**Biologisches Beispiel**

Die im Labor erarbeitete Formulierung (Beispiel 1) wird in einem In Vivo Versuch getestet. Hierzu stehen für Formulierung und Kontrollgruppe je drei Hunde der Rasse Beagle zur Verfügung. Getestet werden die Mikropartikel in einer Spray-Formulierung, die 66 mg verkapselten Wirkstoff in 30 ml wässriger Dispersion enthält und auf den Hund gesprüht wird.

Der Versuchsformulierung werden Fluoreszenzpartikel zugesetzt, die die gleiche Zusammensetzung wie die Verumpartikel haben, es wird lediglich der Wirkstoff gegen einen Fluoreszenzfarbstoff (Uvitex® OB) ausgetauscht. Diese haben keine Wirkung und dienen lediglich einer schnellen und einfachen Visualisierung der Partikel auf dem Fell. So kann mit Hilfe eines Fluoreszenzmikroskops überprüft werden, ob sich noch Partikel auf dem Fell befinden.

Der durchgeführte In Vitro versucht umfasst neun Hunde der Rasse Beagle, sowohl weiblich als auch männlich. Die Tiere sind 21-30 Monate alt und wiegen zwischen 8,8 und 15,5 kg. Die Identifizierung findet über die Ohr Tattoo Nummern statt. Die Hunde sind ohne klinischen Befund, in einem gesunden Zustand und an die Haltungsbedingungen, wie sie während des Versuchs herrschen, gewöhnt. Kommt es zu unerwarteten Reaktionen auf die Versuchsformulierung oder erkrankt ein Tier aus einem anderen Grund, so wird es aus der Studie herausgenommen. Gehalten werden die Hunde in Einzelkäfigen, um Kreuzreaktionen zu vermeiden. Die Hunde werden nach einem gefertigten Schema mit je 25 weiblichen und 25 männlichen Zecken der Gattung *Rhipicephalus sanguineus* (braune Hundezecke) bestückt. Um ein besseres Beißen der Zecken zu gewährleisten, werden die Hunde zu diesem Zweck narkotisiert. Der Zeitplan der Studie ist nachfolgender Tabelle zu entnehmen.

**Tabelle 16 Zeitplan des Vorgehens im Tierversuch**

| Woche | Studientag | Aktivität |
|---|---|---|
| 0 | -2 | - physische Überprüfung zur Aufnahme<br>- Wiegen der Hunde |
| | -1 | - Infestation mit Zecken |
| | 0 | - Zeckenzählung vor Behandlung<br>- Einteilung der Hunde in Behandlungs- und Kontrollgruppe<br>- Behandlung<br>- klin. Überprüfung (vorher, 2 und 4 h nach Behandlung |
| | 1 | - klin. Überprüfung |
| | 2 | - klin. Überprüfung<br>- Zeckenzählung |
| 1 | 5 | - Infestation mit Zecken |
| | 7 | - detaillierte generelle Gesundheitsüberwachung<br>- Zeckenzählung |
| 2 | 12 | - Infestation mit Zecken |
| | 14 | - detaillierte generelle Gesundheitsüberwachung<br>- Zeckenzählung |
| 3 | 21 | - Infestation mit Zecken |
| | 23 | - detaillierte generelle Gesundheitsüberwachung<br>- Zeckenzählung |
| 6 | 40 | - Infestation mit Zecken |
| | 42 | - detaillierte generelle Gesundheitsüberwachung<br>- Zeckenzählung |
| 7 | 48 | - Infestation mit Zecken |
| | 50 | - detaillierte generelle Gesundheitsüberwachung<br>- Zeckenzählung |

(fortgesetzt)

| Woche | Studientag | Aktivität |
|---|---|---|
| 9 | 61 | - Infestation mit Zecken |
| | 63 | - detaillierte generelle Gesundheitsüberwachung<br>- Zeckenzählung |

24 h vor Behandlungsbeginn (Studientag = Study Day = SD -1) werden die Zecken auf die Hunde gesetzt. Kurz vor Beginn des Versuchs (SD 0) werden die auf dem Hund verbliebenen Zecken gezählt und die Zahl notiert. Eine Einteilung der Tiere in Behandlungs- und Kontrollgruppe wird nach der Anzahl der auf den Hunden befindlichen Zecken vorgenommen. In jeder Gruppe sollen bei Versuchsbeginn ungefähr gleich viele Zecken vorliegen, um möglichst gleiche Ausgangsbedingungen zu schaffen. Die Hunde in der Behandlungsgruppe werden mit der Versuchsformulierung am gesamten Körper gleichmäßig eingesprüht. Die Kontrollgruppe wird nicht behandelt. Aus Sicherheitsgründen werden die Hunde 2 und 4 h nach der Applikation auf ihren Gesundheitszustand untersucht. Die Auszählung und Entfernung der noch am Hund befindlichen, lebenden Zecken findet 48 h nach Auftrag der Formulierung statt. Eine Überprüfung der Wirksamkeit erfolgt an SD 2, 7, 23, 50, 63. An den Studientagen SD 2, 7, 14 und 42 werden den Hunden Haarproben entnommen (ca. 100 mg/Probe), die analytisch auf den Wirkstoff Flumethrin untersucht werden. Um eine repräsentative Aussage über den Verbleib von Flumethrinpartikeln auf dem Hund treffen zu können, werden die Fellproben an verschiedenen Körperstellen entnommen. Einzelne Haare werden mikroskopisch untersucht. Im Fluoreszenzmikroskop werden die Placebo Partikel sichtbar, die mit dem Fluoreszenzfarbstoff Uvitex® OB beladen sind. Im Verlauf des Versuchs sinkt die Partikelzahl auf dem Hundehaar, die über elektrostatische Wechselwirkungen auf dem Fell haften. Die fluoreszierenden Partikel haben lediglich eine Indikatorfunktion. Die Verumpartikel können bei dieser Darstellungsweise nicht abgebildet werden.

Die mikroskopischen Aufnahmen dienen einer anschaulichen Darstellungsweise und einer schnellen und einfachen Überprüfung von Partikeln auf dem Haar. Zusätzlich werden die Proben analytisch aufgearbeitet. Dazu werden die Fellproben mit 5 ml Acetonitril überschichtet. Über Nacht wird so der Wirkstoff Flumethrin aus den auf dem Fell verbliebenen Partikeln extrahiert. Ein Teil des Acetonitrils wird filtriert und mittels HPLC-Analyse aufgearbeitet. Die Ergebnisse zeigt Fig. 9.

Aus den Zeckenzahlen auf dem Hund werden das geometrische Mittel und die Effizienz wie folgt berechnet.

$$\bar{x}_{geom} = \sqrt[n]{x_1 \cdot x_2 \cdot x_3 \ldots\ldots \cdot x_n} \qquad \textbf{(Gl. 1)}$$

$$Effizienz \quad \% = \frac{N_2 - N_1}{N_2} \cdot 100 \qquad \textbf{(Gl. 2)}$$

*N1 Geometrisches Mittel der Zeckenzählung der Verumgruppe*

*N2 Geometrisches Mittel der Zeckenzählung der Kontrollgruppe*

Die Anzahl der ausgezählten Zecken an den jeweiligen Studientagen für die einzelnen Hunde (Dog ID = Hunde Identifikationsnummer; die letzten vier Ziffern der Ohrtätowierung) zeigt Tabelle 17.

Tabelle 17 Ergebnisse des Tierversuchs

| Study Group | Dog ID | Zeckenzahlen | | | | | |
|---|---|---|---|---|---|---|---|
| | | SD 0 | SD 2 | SD 7 | SD 23 | SD 50 | SD 63 |
| 12-PMMA 15,0 % Flumethrin | 4641 | 20 | 4 | 1 | 2 | 1 | 7 |
| | 6520 | 14 | 4 | 3 | 5 | 0 | 6 |
| | 8918 | 19 | 6 | 0 | 2 | 1 | 17 |
| | geom. Mittel | | 4,6 | 1 | 2,8 | 0,6 | 9 |
| | Effizienz | | 71,2 | 89,8 | 72,3 | 93,2 | 24,3 |
| Kontroll-gruppe unbehandelt | 6231 | 10 | 10 | 2 | 4 | 9 | 6 |
| | 4596 | 18 | 25 | 37 | 14 | 12 | 27 |
| | 4586 | 20 | 16 | 10 | 17 | 6 | 10 |
| | geom. Mittel | | 15,9 | 9,8 | 10,1 | 8,7 | 11,9 |

SD 0 ist der Tag an dem die Hunde mit der Partikelformulierung eingesprüht werden. An SD 2 werden die noch auf dem Hund befindlichen Zecken ausgezählt. Im weiteren Verlauf des Versuchs kommt es zu weiteren Infestationen der Hunde mit Zecken und einer Auszählung dieser nach 48 h. Ein erneutes Einsprühen der Hunde mit der Versuchsformulierung erfolgt nicht. Die relativ hohen Schwankungen der Effizienz zwischen den Studientagen erklären sich aus der Zahl der verwendeten Versuchstiere und aus der bekannt hohen Varianz biologischer Versuche. Trotzdem zeigt Tabelle 17 deutlich, dass bis SD 50 eine gute Wirksamkeit gegen Zecken festzustellen ist. Die Wirkung lässt erst zwischen SD 50 und 63 deutlich nach. Somit kann mit der erfindungsgemäßen Formulierung des Beispiels 1 eine Wirkungsdauer von 7 Wochen gegen Zecken erreicht werden

Abbildungsverzeichnis:

Fig. 1: Partikelgrößenanalyse in Wasser, Auswertung nach Fraunhofer Beugung
Fig. 2: Rasterelektronenmikroskopische Aufnahme der Partikel 12-PMMA
Fig. 3: Freisetzung Flumethrin aus PMMA Partikeln
Fig. 4: DSC Analyse
Fig. 5: PMMA/PS 60/40
Fig. 6: Placebo - Partikel aus Ethylacetat hergestellt
Fig. 7: DSC Analyse
Fig. 8: PMMA Partikel mit Icaridin beladen
Fig. 9: Änderung des Flumethringehalts auf dem Fell im Verlauf des Versuchs

**Patentansprüche**

**1.** Partikel mit einer Partikelgröße d(v,90) von maximal 10 µm enthaltend

a) ein ungeladenes Polyacrylat und
b) ein kationisches Polyacrylat, das positiv geladene funktionelle Gruppen trägt, wobei die Partikel

c) einen oder mehrere Aktivstoffe enthalten und
d) optional weitere Polymere, Hilfs- oder Zusatzstoffe enthalten können.

**2.** Partikel gemäß Anspruch 1 mit einer Partikelgröße von d(v,90) von 0,1-3 μm.

**3.** Partikel gemäß einem der Ansprüche 1 oder 2 worin das kationische Polyacrylat b) ein quaternisiertes Dialkylaminoalkylmethacrylat-Copolymer ist.

**4.** Partikel gemäß Anspruch 3, worin das kationische Polyacrylat b) ein Copolymer aus Acrylsäure(methyl,ethyl)estern, Methacrylsäure(methyl,ethyl)estern und mit Monochlormethan quaternisierten Dimethylamminoethylestern der Methacrylsäure ist (bekannt unter den Handelsnamen Eudragit RS oder Eudragit RL).

**5.** Partikel gemäß einem der vorstehenden Ansprüche, worin das ungeladene Polyacrylat a) Polymethylmethacrylat ist.

**6.** Partikel gemäß einem der vorstehenden Ansprüche, wobei das Mischungsverhältnis des ungeladenen Polyacrylats a) zu dem kationischen Polyacrylat b) 5:95 m/m bis 95:5 m/m, bevorzugt 70:30 m/m bis 95:5 m/m, besonders bevorzugt 80:20 m/m bis 90:10 m/m beträgt.

**7.** Partikel gemäß einem der vorstehenden Ansprüche, enthaltend, bezogen auf die Masse der Partikel, 0,1 - 50 Gew.-%, bevorzugt 1 - 20 Gew.-% , besonders bevorzugt 5 - 15 Gew.-% Aktivstoffe.

**8.** Partikel gemäß einem der vorstehenden Ansprüche, wobei das ungeladene Polyacrylat ein gewichtsmittleres Molgewicht von 1000 g/mol bis zu 1.000.000 g/mol, vorzugsweise 20.000 bis 600.000g/mol, besonders bevorzugt 50.000 - 150.000 g/mol aufweist.

**9.** Partikel gemäß einem der vorstehenden Ansprüche, enthaltend 0,1-50 Gew.-% bezogen auf die Gesamtmasse der Partikel eines oder mehrerer weiterer Polymere, bevorzugt Polystyrol.

**10.** Partikel gemäß einem der vorstehenden Ansprüche, enthaltend einen oder mehrere Weichmacher, Tenside, Kosolventien, wobei deren summierter Anteil bezogen auf die Gesamtmasse der Mikropartikel 0,1 - 40 Gew.-% , bevorzugt 5 - 30 Gew.-%, besonders bevorzugt 5-20 Gew.-% beträgt.

**11.** Partikel gemäß einem der vorstehenden Ansprüche, enthaltend als Aktivstoff Flumethrin..

**12.** Partikel gemäß einem der vorstehenden Ansprüche, enthaltend als Aktivstoff ein Repellens, bevorzugt Icaridin oder N,N-Diethyl-m-toluamid.

**13.** Verfahren zur Herstellung der Partikel gemäß einem der vorstehenden Ansprüche, mit den folgenden Schritten:

(i) Herstellung einer Lösung der Bestandteile a) bis d) in einem nicht oder nur in geringem Maße mit Wasser mischbaren Lösemittel oder Lösemittelgemisch (1),
(ii) Dispergierung der Lösung (i) in einer wässrigen Phase, optional enthaltend Zusatzstoffe und Lösemittel oder Lösemittelgemisch (1) bis zur Sättigung, zur Erzeugung einer feinen, stabilen Emulsion,
(iii) Entfernung des Lösemittels oder Lösemittelgemischs (1) aus den Emulsionströpfchen durch

- I) Verdampfung ("Solvent Evaporation" Verfahren) unter Erhalt einer wässrigen Suspension oder
- II) Sprühtrocknung unter Erhalt eines trockenen Pulvers.

**14.** Mittel enthaltend Partikel gemäß einem der Ansprüche 1 bis 12.

**15.** Verwendung der Partikel gemäß einem der Ansprüche 1 bis 12 zur Herstellung von Mitteln zur Bekämpfung von Parasiten bei Tieren.

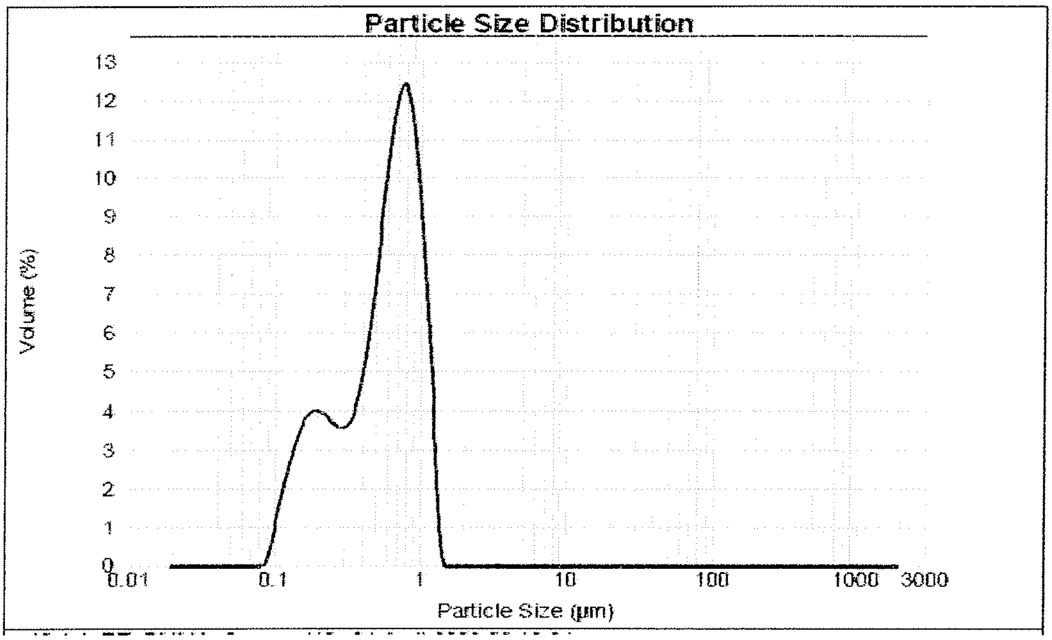
Particle Size Distribution
13
12
11
10
9
8
7
6
5
4
3
2
1
0
Volume (%)
0.01
0.1
1
10
100
1000
3000
Particle Size (µm)

Fig. 1

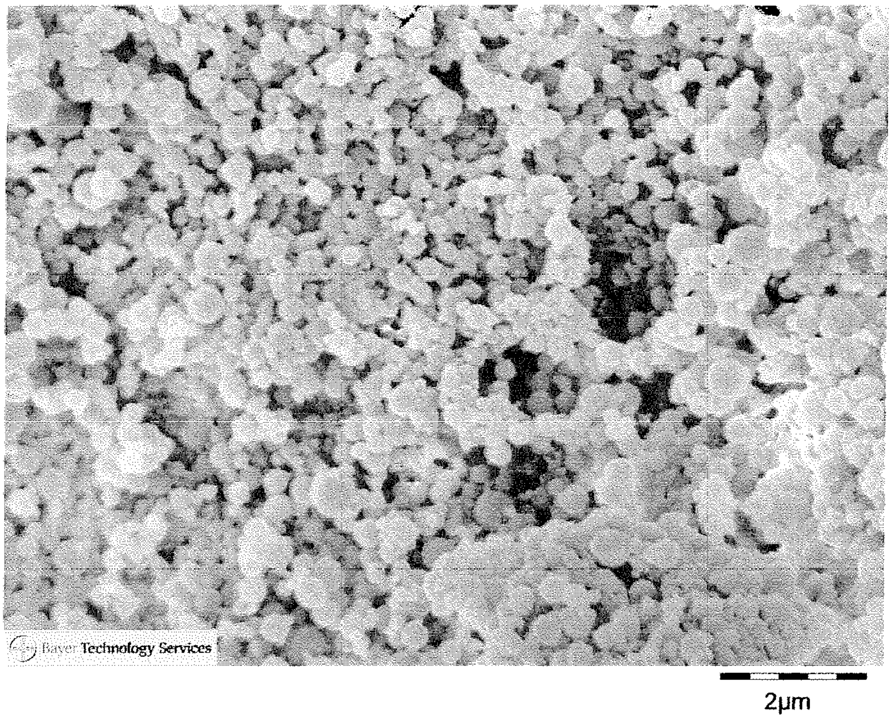
Bayer Technology Services
2µm

Fig. 2

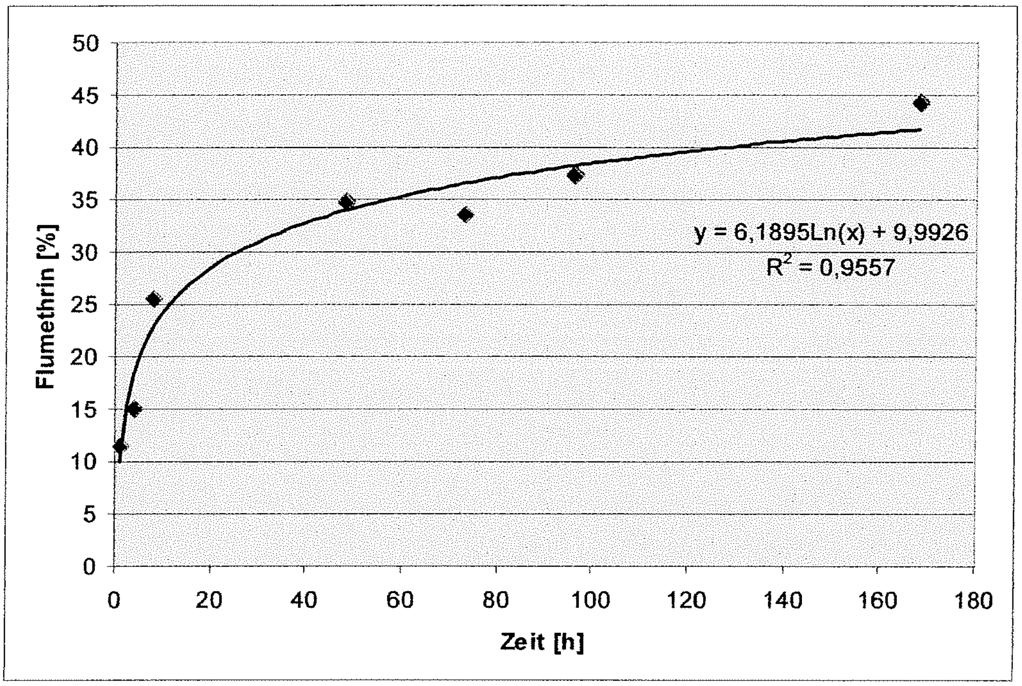

Flumethrin [%]
0
5
10
15
20
25
30
35
40
45
50
Zeit [h]
0
20
40
60
80
100
120
140
160
180
y = 6,1895Ln(x) + 9,9926
R^2 = 0,9557


Fig. 3

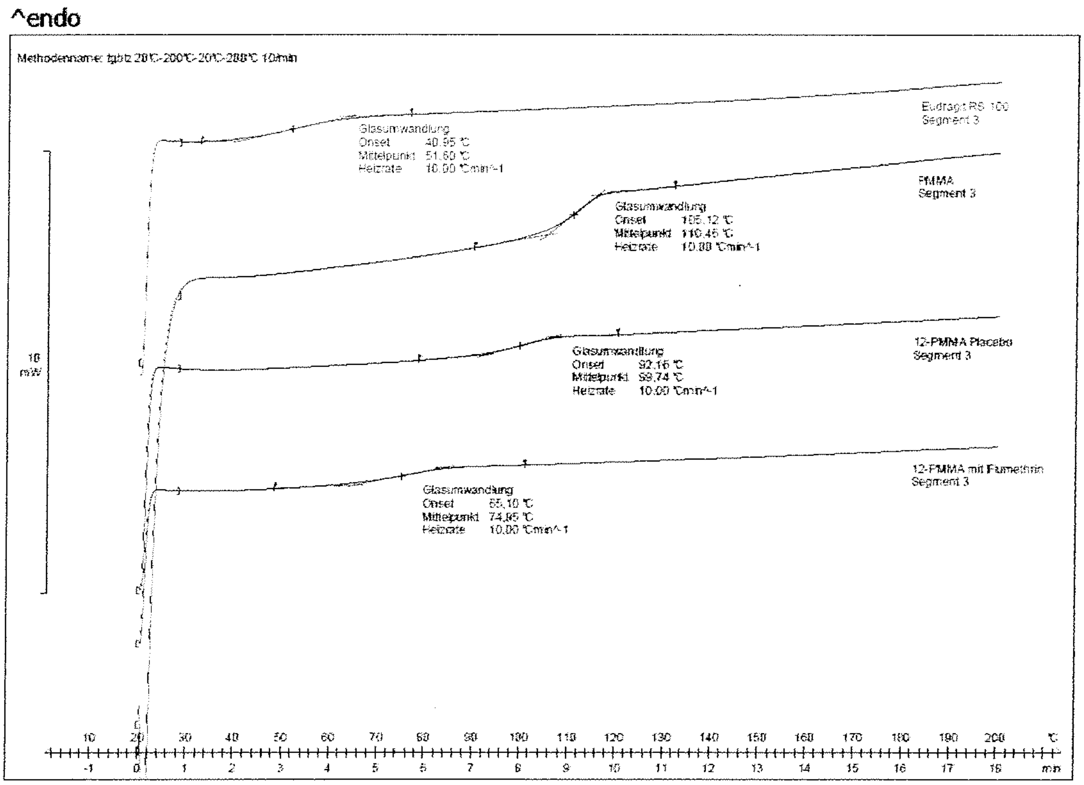

^endo
Eudragit RS 100
Segment 3
Glasumwandlung
Onset 40,95 °C
Mittelpunkt 51,60 °C
Heizrate 10,00 °Cmin^-1
PMMA
Segment 3
Glasumwandlung
Onset 105,12 °C
Mittelpunkt 110,45 °C
Heizrate 10,00 °Cmin^-1
12-PMMA Placebo
Segment 3
Glasumwandlung
Onset 92,16 °C
Mittelpunkt 99,74 °C
Heizrate 10,00 °Cmin^-1
12-PMMA mit Flumethrin
Segment 3
Glasumwandlung
Onset 65,10 °C
Mittelpunkt 74,85 °C
Heizrate 10,00 °Cmin^-1
10
mW
°C
min


Fig. 4

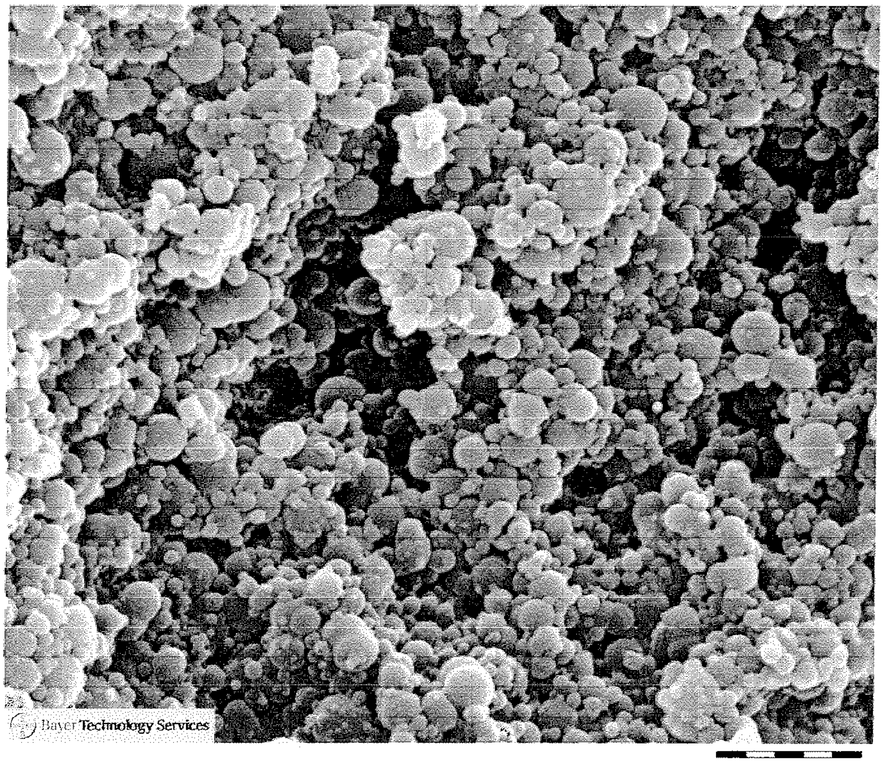
Bayer Technology Services
2µm

Fig. 5

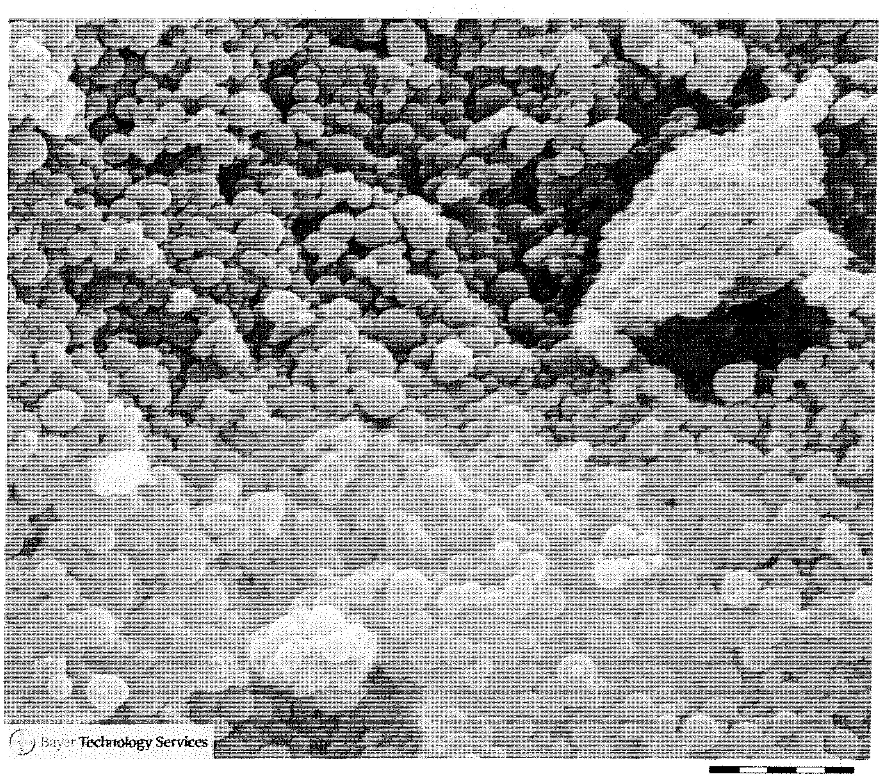
Bayer Technology Services
2µm

Fig. 6

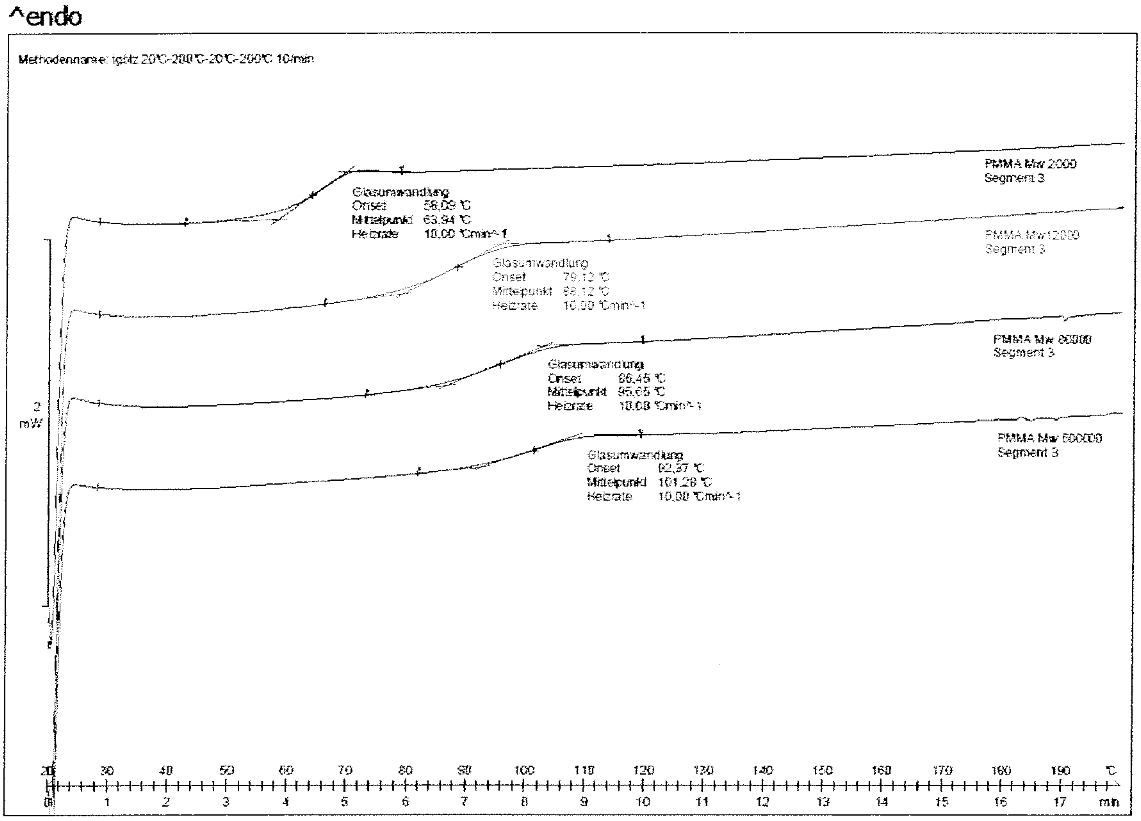
^endo
Methodenname: igbtz 20°C-200°C-20°C-200°C 10/min
Glasumwandlung
Onset 58,09 °C
Mittelpunkt 63,94 °C
Heizrate 10,00 °Cmin^-1
PMMA Mw 2000
Segment 3
Glasumwandlung
Onset 79,12 °C
Mittelpunkt 88,12 °C
Heizrate 10,00 °Cmin^-1
PMMA Mw12000
Segment 3
Glasumwandlung
Onset 86,45 °C
Mittelpunkt 95,65 °C
Heizrate 10,00 °Cmin^-1
PMMA Mw 80000
Segment 3
Glasumwandlung
Onset 92,37 °C
Mittelpunkt 101,28 °C
Heizrate 10,00 °Cmin^-1
PMMA Mw 600000
Segment 3
2
mW
20 30 40 50 60 70 80 90 100 110 120 130 140 150 160 170 180 190 °C
0 1 2 3 4 5 6 7 8 9 10 11 12 13 14 15 16 17 min

**Fig. 7**

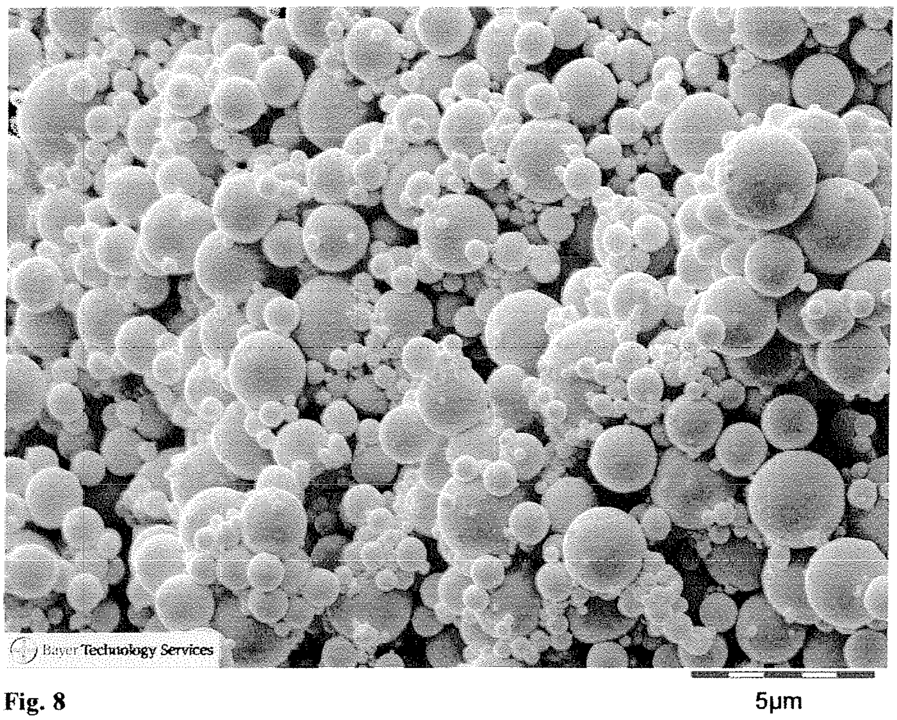
Bayer Technology Services
5µm

**Fig. 8**

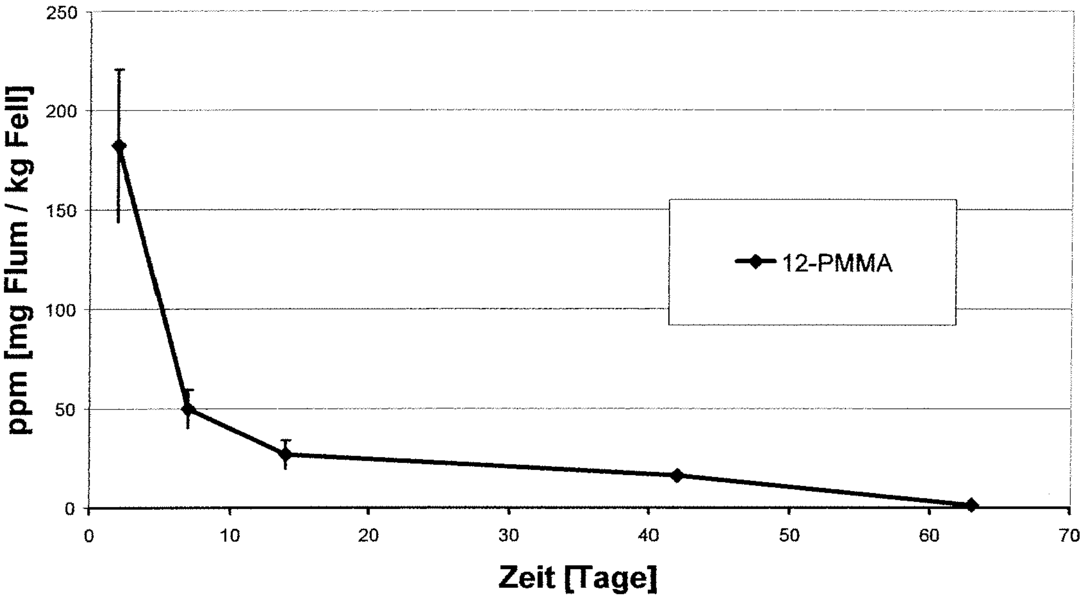
250
200
150
100
50
0
ppm [mg Flum / kg Fell]
12-PMMA
0
10
20
30
40
50
60
70
Zeit [Tage]

Fig. 9

Europäisches Patentamt
European Patent Office
Office européen des brevets

# EUROPÄISCHER RECHERCHENBERICHT

Nummer der Anmeldung
EP 11 17 5537

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 92/01443 A1 (BEECHAM GROUP PLC [GB]) 6. Februar 1992 (1992-02-06)<br>* Anspruch 1; Beispiel 1 *<br>----- | 1-14 | INV.<br>A01N25/24<br>A61K8/81<br>A61Q17/02<br>A61K9/16<br>A61K9/50 |
| X | WO 97/45012 A1 (UNIV BEN GURION [IL]; YISSUM RES DEV CO [IL]; GODWIN EDGAR JAMES [GB];) 4. Dezember 1997 (1997-12-04)<br>* Ansprüche 1,5 *<br>----- | 1-15 | |
| X | WO 01/35933 A2 (TAGRA BIOTECHNOLOGIES LTD [IL]; BABTSOV VLADIMIR [IL]; SHAPIRO YURY [I) 25. Mai 2001 (2001-05-25)<br>* Anspruch 1 *<br>----- | 13,14 | |
| A | EP 0 848 950 A2 (ROEHM GMBH [DE] ROEHM GMBH) 24. Juni 1998 (1998-06-24)<br>* Anspruch 1 *<br>----- | 1-14 | |
| A | WO 01/87243 A1 (UNILEVER PLC [GB]; UNILEVER NV [NL]; LEVER HINDUSTAN LTD [IN]) 22. November 2001 (2001-11-22)<br>* Anspruch 1 *<br>----- | 1-14 | RECHERCHIERTE SACHGEBIETE (IPC)<br>A01N<br>A61K<br>A61Q |

Der vorliegende Recherchenbericht wurde für alle Patentansprüche erstellt

1

| Recherchenort | Abschlußdatum der Recherche | Prüfer |
|---|---|---|
| München | 22. Dezember 2011 | Giese, Hans-Hermann |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 11 17 5537

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

22-12-2011

| Im Recherchenbericht angeführtes Patentdokument | Datum der Veröffentlichung | Mitglied(er) der Patentfamilie | Datum der Veröffentlichung |
|---|---|---|---|
| WO 9201443 A1 | 06-02-1992 | CA 2087447 A1 | 19-01-1992 |
| | | EP 0539428 A1 | 05-05-1993 |
| | | IE 912484 A1 | 29-01-1992 |
| | | JP H05509088 A | 16-12-1993 |
| | | WO 9201443 A1 | 06-02-1992 |
| | | ZA 9105546 A | 29-07-1992 |
| WO 9745012 A1 | 04-12-1997 | AU 2967797 A | 05-01-1998 |
| | | IL 118439 A | 29-06-2000 |
| | | WO 9745012 A1 | 04-12-1997 |
| WO 0135933 A2 | 25-05-2001 | AU 774354 B2 | 24-06-2004 |
| | | AU 1298601 A | 30-05-2001 |
| | | CA 2389688 A1 | 25-05-2001 |
| | | CN 1391467 A | 15-01-2003 |
| | | EP 1231904 A2 | 21-08-2002 |
| | | EP 2047844 A2 | 15-04-2009 |
| | | GB 2356386 A | 23-05-2001 |
| | | JP 2003514008 A | 15-04-2003 |
| | | MX PA02004919 A | 14-10-2003 |
| | | US 6932984 B1 | 23-08-2005 |
| | | WO 0135933 A2 | 25-05-2001 |
| | | ZA 200203697 A | 09-05-2003 |
| EP 0848950 A2 | 24-06-1998 | DE 19653606 A1 | 25-06-1998 |
| | | EP 0848950 A2 | 24-06-1998 |
| | | ES 2183069 T3 | 16-03-2003 |
| | | JP 10182440 A | 07-07-1998 |
| | | US 5993849 A | 30-11-1999 |
| WO 0187243 A1 | 22-11-2001 | AR 029093 A1 | 04-06-2003 |
| | | AU 6392601 A | 26-11-2001 |
| | | BR 0110885 A | 10-06-2003 |
| | | EP 1282389 A1 | 12-02-2003 |
| | | JP 2003533456 A | 11-11-2003 |
| | | US 2001055579 A1 | 27-12-2001 |
| | | WO 0187243 A1 | 22-11-2001 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1092417 A
- JP 3077820 A
- WO 02060417 A
- US 5438076 A
- EP 0404558 A
- JP 63130541 A
- WO 0187243 A
- WO 9738667 A
- US 5753264 A
- US 0142828 A
- FR 2801811
- WO 0135933 A
- EP 1407753 A
- EP 1407754 A
- WO 02060399 A
- EP 0369741 A
- WO 9828339 A
- WO 9201443 A
- WO 09056280 A


### In der Beschreibung aufgeführte Nicht-Patentliteratur


- Evonik Industries: Eudragit® Application Guidelines. Evonik Industries AG, 2008
- Evonik Industries: Eudragit Application Guidelines. Evonik Industries AG, 2008
- *Drug Development and Industrial Pharmacy,* 1990, vol. 16 (13), 2057-2075
- *Journal of Controlled Release,* 1991, vol. 16, 311-318
- **GOTO S. et al.** *J. Microencapsulation,* 1986, vol. 3 (4), 293-304
- **GOTO S. et al.** *J Microencapsulation,* 1988, vol. 5 (4), 343-360
- **SATTURWAR P.M. et al.** *Microencapsulation,* 2002, vol. 19 (4), 407-413